(19) Europäisches Patentamt
European Patent Office
Office européen des brevets

(11) **EP 4 414 689 A1**

(12) **EUROPEAN PATENT APPLICATION**
published in accordance with Art. 153(4) EPC

(43) Date of publication:
**14.08.2024 Bulletin 2024/33**

(21) Application number: **22887086.1**

(22) Date of filing: **26.10.2022**

(51) International Patent Classification (IPC):
**G01N 21/3581** (2014.01)

(52) Cooperative Patent Classification (CPC):
**G01N 21/3581**

(86) International application number:
**PCT/JP2022/040011**

(87) International publication number:
**WO 2023/074761 (04.05.2023 Gazette 2023/18)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **27.10.2021 JP 2021175526**

(71) Applicant: **Hamamatsu Photonics K.K.
Hamamatsu-shi, Shizuoka 435-8558 (JP)**

(72) Inventors:
• **TAKAHASHI, Kazuhiro
Hamamatsu-shi, Shizuoka 435-8558 (JP)**
• **AKIYAMA, Kouichiro
Hamamatsu-shi, Shizuoka 435-8558 (JP)**
• **SATOZONO, Hiroshi
Hamamatsu-shi, Shizuoka 435-8558 (JP)**

(74) Representative: **Boult Wade Tennant LLP
Salisbury Square House
8 Salisbury Square
London EC4Y 8AP (GB)**

(54) **CRYSTAL FORM DETERMINATION METHOD**

(57)     A crystal form determination method includes a first process of preparing a measurement target object; a second process of entering a terahertz wave to the measurement target object and detecting the terahertz wave from the measurement target object to acquire a plurality of detection results corresponding to a plurality of times separated from each other; and a third process of determining a type of crystal form contained in the measurement target object based on a plurality of frequency characteristics calculated from the plurality of detection results. In the third process, when a frequency characteristic having a third peak at a third frequency different from a first frequency related to a first peak corresponding to an anhydride and a second frequency related to a second peak corresponding to a hydrate exists among the plurality of frequency characteristics, determination is made that the crystal form different from the anhydride and the hydrate is contained in the measurement target object.

**Fig.6**

**Description**

**Technical Field**

The present disclosure relates to a crystal form determination method.

**Background Art**

Conventionally, for example, a method for researching physicochemical properties of a measurement target object is known as information on the measurement target object (see e.g., Non-Patent Document 1). In such a method, the measurement target object is analyzed in detail by acquiring various pieces of information on anhydrides and hydrates contained in the measurement target object, for example, by an X-ray diffraction method or the like.

**Citation List**

**Non Patent Literature**

Non Patent Literature 1: Dori E. Braun and 3 others, "The Complexity of Hydration of Phloroglucinol: A Comprehensive Structural and Thermodynamic Characterization", THE JOURNAL OF PHYSICAL CHEMISTRY B, 116, 2012, p. 3961 to 3972

**Summary of Invention**

**Technical Problem**

However, in order to perform detailed analysis on the measurement target object, it is insufficient with only the information as described above, and acquisition of more detailed information on the measurement target object is required.
An object of the present disclosure is to provide a crystal form determination method capable of acquiring detailed information on a measurement target object.

**Solution to Problem**

A crystal form determination method according to one aspect of the present disclosure is [1] "a crystal form determination method including a first process of preparing a measurement target object; a second process of entering a terahertz wave with respect to the measurement target object and detecting the terahertz wave from the measurement target object to acquire a plurality of detection results corresponding to a plurality of times separated from each other; and a third process of determining a type of crystal form contained in the measurement target object based on a plurality of frequency characteristics calculated from the plurality of detection results; wherein in the third process, when a frequency characteristic having a third peak at a third frequency different from a first frequency related to a first peak corresponding to an anhydride and a second frequency related to a second peak corresponding to a hydrate exists among the plurality of frequency characteristics, determination is made that a crystal form different from the anhydride and the hydrate is contained in the measurement target object".
In the crystal form determination method described in [1], when a frequency characteristic having a third peak at a third frequency different from the first frequency and the second frequency exists among a plurality of frequency characteristics, determination is made that a crystal form different from an anhydride and a hydrate is contained in the measurement target object. As a result, whether or not the crystal form different from the anhydride and the hydrate exists can be determined for a predetermined objects-to-be-measured, and furthermore, various measurement target object can be classified into the measurement target object in which the crystal form different from the anhydride and the hydrate exists and the measurement target object in which the crystal form different from the anhydride and the hydrate does not exist. Therefore, according to the crystal form determination method, detailed information on the measurement target object can be acquired.
The crystal form determination method according to one aspect of the present disclosure may be [2] "the crystal form determination method according to [1], wherein in the third process, when a frequency characteristic having the first peak at the first frequency exists among the plurality of frequency characteristics, determination is made that the anhydride is contained in the measurement target object". Thus, not only the crystal form different from the anhydride and the hydrate but also whether or not the anhydride is contained in the measurement target object can be determined. Therefore, more detailed information about the measurement target object can be acquired.
The crystal form determination method according to one aspect of the present disclosure may be [3] "the crystal form determination method according to [1] or [2], wherein in the third process, when a frequency characteristic having the second peak at the second frequency exists among the plurality of frequency characteristics, determination is made that the hydrate is contained in the measurement target object". This makes it possible to determine not only the crystal form different from the anhydride and the hydrate but also whether or not the hydrate is contained in the measurement target object. Therefore, more detailed information about the measurement target object can be acquired.

The crystal form determination method according to one aspect of the present disclosure may be [4] "the crystal form determination method according to any one of [1] to [3], wherein in the third process, when at least two frequency characteristics having peaks at frequencies different from each other exist among the plurality of frequency characteristics, determination is made that a hydration reaction of an anhydride or a dehydration reaction of a hydrate proceeded in the measurement target object". This makes it possible to determine whether or not the hydration reaction of the anhydride or the dehydration reaction of the hydrate proceeded in the measurement target object. Therefore, more detailed information about the measurement target object can be acquired.

A crystal form determination method according to another aspect of the present disclosure is [5] "a crystal form determination method including a first process of preparing a measurement target object containing phloroglucinol; a second process of entering a terahertz wave with respect to the measurement target object and detecting the terahertz wave from the measurement target object to acquire a detection result related to the measurement target object; and a third process of determining a type of crystal form contained in the measurement target object based on a frequency characteristic calculated from the detection result; wherein in the third process, when a frequency characteristic has a peak in 2.65 THz to 2.95 THz, determination is made that a crystal form different from the anhydride and the hydrate is contained in the measurement target object."

In the crystal form determination method described in [5], when the frequency characteristic has a peak in 2.65 THz to 2.95 THz, determination is made that a crystal form different from an anhydride and a hydrate is contained in the measurement target object. This makes it possible to determine whether or not the measurement target object containing phloroglucinol contains a crystal form different from the anhydride and the hydrate. Therefore, according to the crystal form determination method, detailed information on the measurement target object can be acquired.

The crystal form determination method according to another aspect of the present disclosure may be [6] "the crystal form determination method according to [5], wherein in the third process, when the frequency characteristic has a peak in 1.7 THz to 1.9 THz or 3.25 THz to 3.45 THz, determination is made that the anhydride is contained in the measurement target object". This makes it possible to determine not only the crystal form different from the anhydride and the hydrate but also whether or not the anhydride is contained in the measurement target object. Therefore, more detailed information about the measurement target object can be acquired.

The crystal form determination method according to another aspect of the present disclosure may be [7] "the crystal form determination method according to [5] or [6], wherein in the third process, when the frequency characteristic has a peak in 3.1 THz to 3.25 THz, determination is made that the hydrate is contained in the measurement target object". This makes it possible to determine not only the crystal form different from the anhydride and the hydrate but also whether or not the hydrate is contained in the measurement target object. Therefore, more detailed information about the measurement target object can be acquired.

The crystal form determination method according to another aspect of the present disclosure may be [8] "the crystal form determination method according to any one of [5] to [7], wherein in the second process, the terahertz wave of a broadband wavelength having a frequency in a range of at least 2.65 THz to 2.95 THz is entered to the measurement target object". As a result, the terahertz wave T of a broadband wavelength can be collectively entered to the measurement target object, and information on the measurement target object can be efficiently acquired.

The crystal form determination method according to another aspect of the present disclosure may be [9] "the crystal form determination method according to any one of [5] to [7], wherein in the second process, the terahertz wave of a single wavelength having at least one frequency selected from a range of at least 2.65 THz to 2.95 THz is entered to the measurement target object". As a result, information on the measurement object can be acquired with a simple configuration.

### Advantageous Effects of Invention

According to the present disclosure, a crystal form determination method capable of acquiring detailed information on a measurement target object can be provided.

### Brief Description of Drawings

FIG. 1 is a configuration diagram of a spectroscopic device according to a first embodiment.

FIG. 2 is a cross-sectional view of a peripheral structure of an arrangement unit illustrated in FIG. 1.

FIG. 3 is a flowchart of a crystal form determination method according to the first embodiment.

FIG. 4 is a graph illustrating frequency characteristics of an anhydride and a hydrate.

FIG. 5 is a diagram illustrating a temporal change in the frequency characteristic of a measurement target object.

FIG. 6 is a graph illustrating a comparison between the frequency characteristic of a measurement target object and the frequency characteristic of an anhydride, and a comparison between the frequency characteristic of a measurement target object and the frequency characteristic of a hydrate.

FIG. 7 is a table illustrating a moisture content of a hydrate.

FIG. 8 is a table illustrating a moisture content of an intermediate.

FIG. 9 is a graph illustrating a comparison between the frequency characteristic of a measurement target object and the frequency characteristic of an anhydride, and a comparison between the frequency characteristic of a measurement target object and the frequency characteristic of a hydrate.

FIG. 10 is a diagram illustrating second derivatives of the frequency characteristic in FIG. 9.

FIG. 11 is a graph illustrating a comparison between the frequency characteristic of a measurement target object and the frequency characteristic of an anhydride, and a comparison between the frequency characteristic of a measurement target object and the frequency characteristic of a hydrate.

FIG. 12 is a diagram illustrating second derivatives of the frequency characteristic in FIG. 11.

FIG. 13 is a flowchart of a crystal form determination method according to the second embodiment.

FIG. 14 is a graph illustrating frequency characteristics of an anhydride, a hydrate, and an intermediate.

FIG. 15 is a table showing frequencies related to peaks corresponding to each of the anhydride, the hydrate, and the intermediate.

FIG. 16 is a view illustrating a comparison between a frequency characteristic of a measurement target object and a frequency characteristic of an intermediate.

FIG. 17 is a configuration diagram of a spectroscopic device according to a modified example.

FIG. 18 is a view illustrating a crystal form determination method in a case where the spectroscopic device illustrated in FIG. 17 is used.

FIG. 19 is a view illustrating a crystal form determination method according to a modified example.

FIG. 20 is a flowchart for determining the proceeding of a hydration reaction or a dehydration reaction.

## Description of Embodiments

Hereinafter, embodiments of the present disclosure will be described in detail with reference to the drawings. The same or corresponding parts in the respective drawings are denoted with the same reference signs, and repetitive descriptions will be omitted.

[First Embodiment] [Spectroscopic Device]

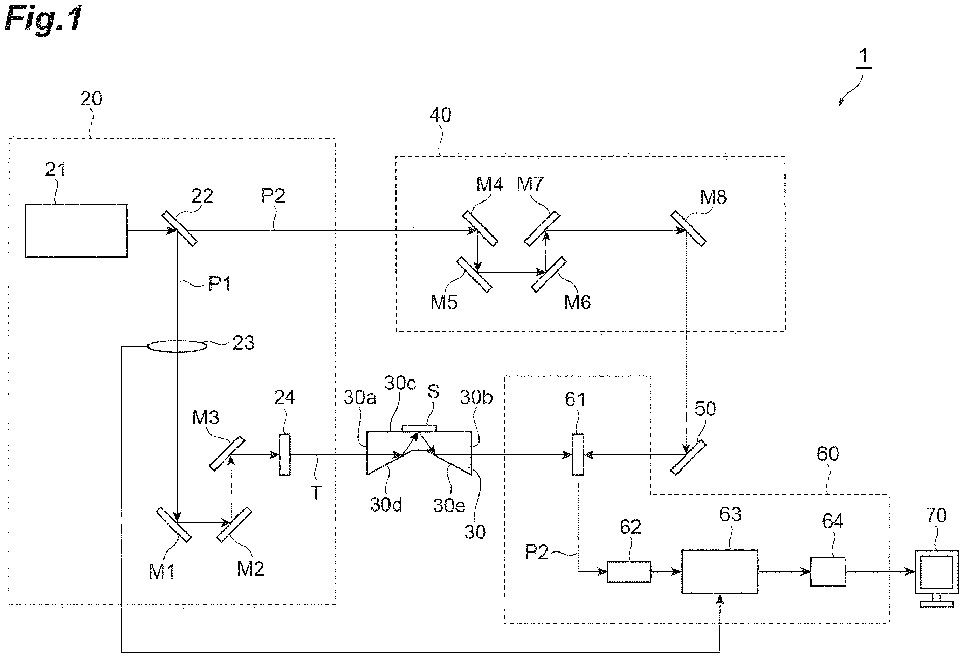

As illustrated in FIG. 1, the spectroscopic device 1 includes an output unit 20, an arrangement unit 30, an adjustment unit 40, a reflection unit 50, a detection unit 60, and a processing unit 70. The spectroscopic device 1 is a device for performing an attenuated total reflection spectroscopy (ATR) using a terahertz wave.

The output unit 20 outputs the terahertz wave T. Specifically, the output unit 20 includes a light source 21, a branching portion 22, a chopper 23, a plurality of mirrors M1 to M3, and a terahertz wave generation element 24. The light source 21 outputs light by pulse oscillation. The light source 21 outputs, for example, a pulsed laser light having a pulse width of about femtosecond. That is, the light source 21 is a femtosecond pulsed laser light source.

The branching portion 22 is, for example, a beam splitter or the like. The branching portion 22 branches the light output from the light source 21 into a pump light P1 and a probe light P2. The chopper 23 alternately repeats passing and shielding of the pump light P1 output from the branching portion 22 at a constant cycle.

Each of the mirrors M1 to M3 sequentially reflects the pump light P1 that passed through the chopper 23. The pump light P1 that passed through the chopper 23 is sequentially reflected by the mirrors M1 to M3, and then enters the terahertz wave generation element 24. Note that, hereinafter, the optical system of the pump light P1 from the branching portion 22 to the terahertz wave generation element 24 is referred to as a "pump optical system".

The terahertz wave generation element 24 outputs the terahertz wave T when the pump light P1 reflected by the mirror M3 is entered. The terahertz wave generation element 24 includes, for example, a non-linear optical crystal (e.g., ZnTe), a photoconductive antenna element (e.g., an optical switch using GaAs), a semiconductor (e.g., InAs), or a superconductor. In a case where the terahertz wave generation element 24 includes the non-linear optical crystal, the terahertz wave generation element 24 generates the terahertz wave T by the non-linear optical phenomenon generated with the entering of the pump light P1.

The terahertz wave T has an intermediate property between an optical wave and a radio wave. The terahertz wave T is an electromagnetic wave having a frequency corresponding to an intermediate region between the optical wave and the radio wave. The terahertz wave T has a frequency of about 0.01 THz to 100 THz. The terahertz wave T is generated at a constant repetition cycle and has a pulse width of about several picoseconds. That is, the terahertz wave generation element 24 generates a pulse light train including a plurality of terahertz waves T arranged at predetermined time intervals (pulse intervals). Note that, hereinafter, an optical system of the terahertz wave T from the terahertz wave generation element 24 to a detector 61 to be described later is referred to as a "terahertz wave optical system".

The arrangement unit 30 is, for example, a so-called aberration-free prism or the like. The arrangement unit 30 includes an incident surface 30a, an exit surface 30b, a reflection surface 30c, a first sub-reflection surface 30d, and a second sub-reflection surface 30e. The incident surface 30a and the exit surface 30b are parallel to each other. The reflection surface 30c is perpendicular to incident surface 30a and exit surface 30b. The measurement target object S is disposed on the reflection surface 30c. The first sub-reflection surface 30d and the second sub-reflection surface 30e are surfaces of the arrangement unit 30 on the side opposite to the reflection surface 30c, and form a recessed portion. A surface configured by the first sub-reflection surface 30d and the second sub-reflection surface 30e is recessed toward the reflection surface 30c.

The arrangement unit 30 is transparent to the terahertz wave T output from the terahertz wave generation element 24. The index of refraction of the arrangement unit 30 is higher than the index of refraction of the measurement target object S. The material of the arrangement unit 30 is, for example, silicon.

The terahertz wave T that entered the incident surface 30a of the arrangement unit 30 is sequentially reflected by the first sub-reflection surface 30d, the reflection surface 30c, and the second sub-reflection surface 30e, and then output to the outside from the exit surface 30b. Information of the terahertz wave band regarding the measurement target object S can be acquired by detecting the attenuation reflectance of the evanescent wave seeping when the terahertz wave T is totally reflected at the reflection surface 30c.

The measurement target object S contains, for example, phloroglucinol (phG). In the measurement target object S, hydration transfer reaction may occur. In the measurement target object S, the anhydride of phloroglucinol is transferred to the hydrate of phloroglucinol. Note that, for example, a dispersant or the like may be mixed with the measurement target object S.

The adjustment unit 40 includes a plurality of mirrors M4 to M8. The probe light P2 output from the branching portion 22 is sequentially reflected by the mirrors M4 to M8, further reflected by the reflection unit 50, and then entered to the detector 61. The reflection unit 50 is a mirror. Note that hereinafter, the optical system of the probe light P2 from the branching portion 22 to the detector 61 is referred to as a "probe optical system".

In the adjustment unit 40, the optical path length between the mirror M4 and the mirror M5 and the optical path length between the mirror M6 and the mirror M7 are adjusted by moving the mirrors M5 and M6. The optical path length of the probe optical system is thereby adjusted. The adjustment unit 40 adjusts a difference between "the optical path length obtained by adding the optical path length of the terahertz wave optical system from the terahertz wave generation element 24 to the detector 61 to the optical path length of the pump optical system from the branching portion 22 to the terahertz wave generation element 24" and "the optical path length of the probe optical system from the branching portion 22 to the detector 61".

The detection unit 60 detects the terahertz wave T output from the arrangement unit 30. Specifically, the detection unit 60 includes a detector 61, an I/V conversion amplifier 62, a lock-in amplifier 63, and an A/D converter 64. When the terahertz wave T output from the arrangement unit 30 and the probe light P2 reflected by the reflection unit 50 enter the detector 61, the detector 61 detects a correlation between the terahertz wave T and the probe light P2.

Specifically, the detector 61 includes a photoconductive antenna and the like. When the probe light P2 enters the detector 61, photocarriers are generated in the detector 61. When the terahertz wave T enters the detector 61 in which the photocarriers are generated, the photocarriers flow according to the electric field of the terahertz wave T, and as a result, the photocarriers are output from the detector 61 as a current. The amount of current output from the detector 61 depends on the electric field intensity of the terahertz wave T.

The current output from the detector 61 is input to the I/V conversion amplifier 62. After converting the current output from the detector 61 into a voltage, the I/V conversion amplifier 62 amplifies the voltage and outputs the voltage to the lock-in amplifier 63. The lock-in amplifier 63 synchronously detects the electric signal output from the I/V conversion amplifier 62 at a repetition frequency of passing and shielding of the pump light P1 in the chopper 23. The A/D converter 64 converts the analog signal from the lock-in amplifier 63 into a digital signal. The signal output from the lock-in amplifier 63 has a value that depends on the electric field intensity of the terahertz wave T. In this manner, the detection unit 60 detects the electric field amplitude of the terahertz wave T by detecting the correlation between the terahertz wave T and the probe light P2.

When the optical path length of the probe optical system is adjusted by adjusting the optical path length between the mirror M4 and the mirror M5 and the optical path length between the mirror M6 and the mirror M7 in the adjustment unit 40, the difference between the timings of the terahertz wave T and the probe light P2 input to the detector 61 is adjusted. As described above, in general, the pulse width of the terahertz wave T is about picoseconds, whereas the pulse width of the probe light P2 is about femtoseconds. That is, the pulse width of the probe light P2 is narrower than the terahertz wave T by several digits. From this, the time waveform (hereinafter, referred to as "electric field waveform") of the electric field amplitude of the terahertz wave T is obtained by sweeping the incident timing of the probe light P2 to the detector 61 by the adjustment unit 40. Hereinafter, acquiring the electric field waveform by such a method is simply referred to as "acquiring the electric field waveform".

When the incident timing of the probe light P2 is swept once, an electric field waveform of one terahertz wave T corresponding to a predetermined time is obtained. In the present embodiment, the adjustment unit 40 sweeps the incident timing of the probe light P2 to the detector 61 over a plurality of times. As a result, a plurality of electric field waveforms are obtained. That is, the detection unit 60 acquires data including a plurality of electric field waveforms (detection results) corresponding to each of a plurality of times separated from each other.

The processing unit 70 acquires information on the measurement target object S based on the plurality of electric field waveforms acquired by the detection unit 60. Specifically, the processing unit 70 calculates a frequency characteristic corresponding to each electric field waveform based on the signal output from the A/D converter 64. The frequency characteristic refers to an optical characteristic with respect to a frequency. The optical characteristics include light absorbency, light reflectivity, light transmittance, or the like. The frequency characteristic is, for example, an absorption spectrum. The processing unit 70 acquires information on the measurement target object S based on each frequency characteristic. Thus, the spectroscopic device 1 measures the temporal change of the measurement target object S. The processing unit 70 includes a central processing unit (CPU), a read only memory (ROM), a random access memory (RAM), and the like.

[Peripheral Structure of Arrangement Unit]

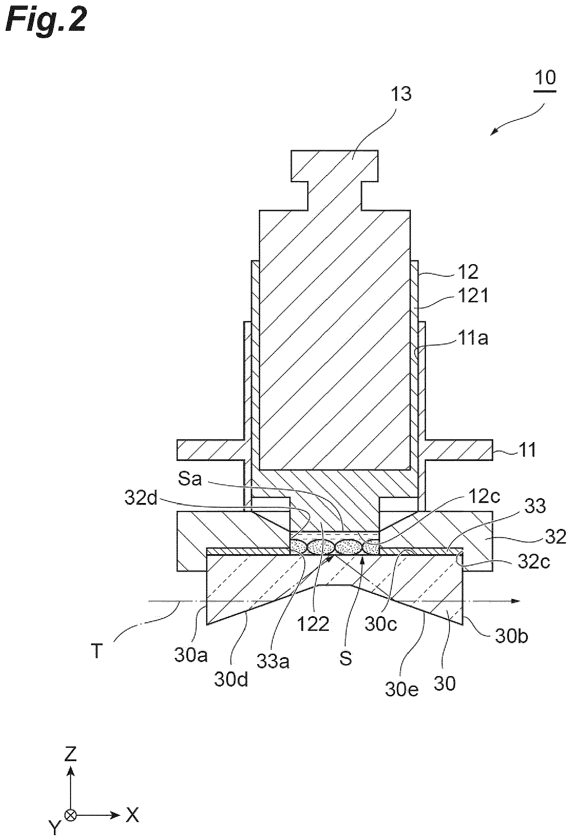

As illustrated in FIG. 2, the spectroscopic device 1 further includes a frame body 32, a sheet 33, and a pressure applying device 10 as a peripheral structure of the arrangement unit 30. In FIG. 1, illustration thereof is omitted.

A recessed portion 32c is formed in the frame body 32. The frame body 32 is disposed on the reflection surface 30c such that a part of the arrangement unit 30 including the reflection surface 30c and the sheet 33 are located in the recessed portion 32c. A through hole 32d and a through hole 33a are formed in the frame body 32 and the sheet 33, respectively. Each of the through hole 32d and through hole 33a has, for example, a circular shape when viewed from the Z-axis direction. The measurement target object S is disposed on the reflection surface 30c on the inner side of the through hole 32d and the through hole 33a. The shape of each of the through hole 32d and the through hole 33a is not limited. Each of the through hole 32d and through hole 33a has, for example, a rectangular shape when viewed from the Z-axis direction.

The pressure applying device 10 includes a supporting portion 11, a contact portion 12, and a biasing portion 13. The supporting portion 11 includes a tubular body extending along the Z-axis direction. The tubular body of the supporting portion 11 has, for example, a cylindrical shape. The contact portion 12 includes a main body part 121 and a protruding part 122. The main body part 121 has a tubular shape with a bottom. The main body part 121 has, for example, a cylindrical shape. The main body part 121 is disposed on the inner side of the supporting portion 11 so as to be slidable with respect to the inner surface 11a of the supporting portion 11. The protruding part 122 protrudes from the bottom of the main body part 121. The protruding part 122 has, for example, a circular column shape. The protruding part 122 enters the through hole 32d, and the distal end face 12c of the protruding part 122 comes into contact with the surface Sa of the measurement target object S. The biasing portion 13 has, for example, a circular column shape. The biasing portion 13 is disposed in an internal space formed in the main body part 121. The biasing portion 13 is, for example, a weight having a constant weight. The biasing portion 13 applies a load corresponding to the weight of the biasing portion 13 to the contact portion 12 along the Z-axis direction. The load is transmitted to the surface Sa of the measurement target object S through the protruding part 122.

The shapes of the supporting portion 11, the contact portion 12, and the biasing portion 13 are not limited. The internal space of the supporting portion 11 may have, for example, a rectangular parallelepiped shape. The internal space of the main body part 121 of the contact portion 12 may have, for example, a rectangular parallelepiped shape. The protruding part 122 of the contact portion 12 may have, for example, a rectangular parallelepiped shape. The biasing portion 13 may have, for example, a rectangular parallelepiped shape.

The pressure applying device 10 is configured to be able to adjust the magnitude of the pressure applied to the measurement target object S. In the pressure applying device 10, for example, change can be made to a weight having a different weight as the biasing portion 13. In the present embodiment, the pressure applying device 10 applies a substantially constant pressure to the measurement target object S. Specifically, as described above, the spread of the measurement target object S along the direction parallel to the reflection surface 30c is restricted by the frame body 32. Therefore, when the load by the biasing portion 13 is transmitted to the measurement target object S through the contact portion 12, the measurement target object S is pressed on the inner side of the frame body 32. Accordingly, pressure is applied to the measurement target object S. Here, since the biasing portion 13 is a weight having a constant weight, a constant load is transmitted to the measurement target object S. Therefore, a constant pressure is applied to the measurement target object S. Note that "applying a substantially constant pressure" means applying a pressure within a range of ± 5% with respect to the reference value.

[Crystal Form Determination Method]

Next, a crystal form determination method according to the first embodiment using the spectroscopic device 1 will be described. In the present embodiment, the type of crystal form was determined for each of the measurement target object S containing phloroglucinol, the measurement target object S containing aminophylline (AP), and the measurement target object S containing theophylline (TP).

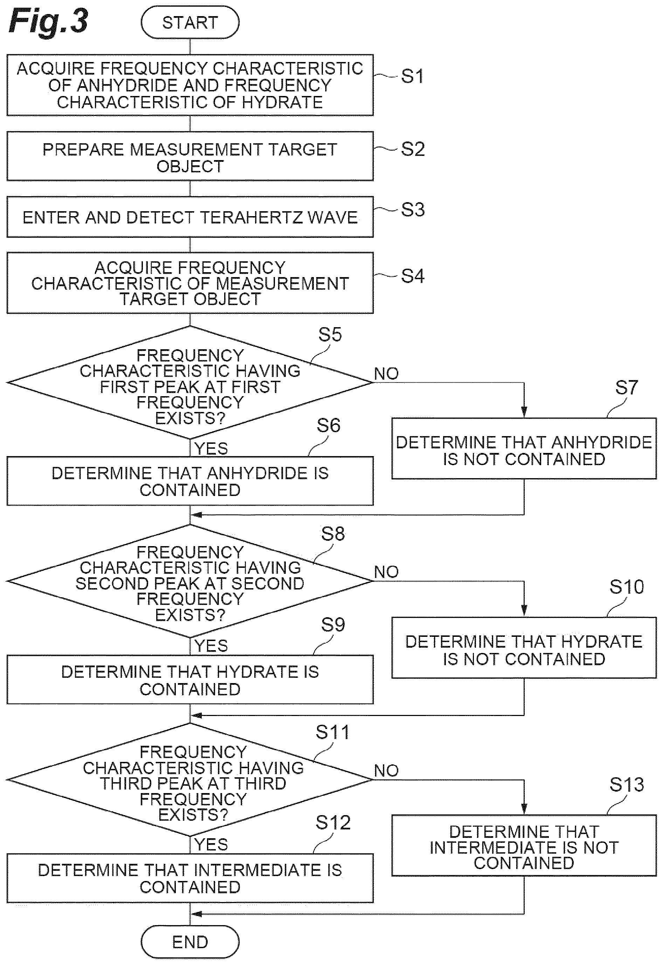

First, the measurement target object S containing phloroglucinol will be described. As illustrated in FIG. 3, first, frequency characteristics of an anhydride of phloroglucinol (hereinafter, referred to as "anhydride") and frequency characteristics of a hydrate of phloroglucinol (hereinafter, referred to as "hydrate") are acquired (step S 1). Specifically, the reference electric field waveform is acquired by causing the terahertz wave T to enter the incident surface 30a in a state where the measurement target object S is not arranged on the reflection surface 30c.

Subsequently, an anhydride prepared in advance is arranged on the reflection surface 30c. Then, an electric field waveform of the anhydride is acquired. Then, the frequency characteristic of the anhydride is calculated based on the reference electric field waveform and the electric field waveform of the anhydride. Subsequently, after the anhydride is removed from the reflection surface 30c, a hydrate prepared in advance is arranged on the reflection surface 30c. In the present embodiment, the hydrate of phloroglucinol is a dihydrate. Then, an electric field waveform of the anhydride is acquired. Subsequently, the frequency characteristic of the hydrate is calculated based on the reference electric field waveform and the electric field waveform of the hydrate.

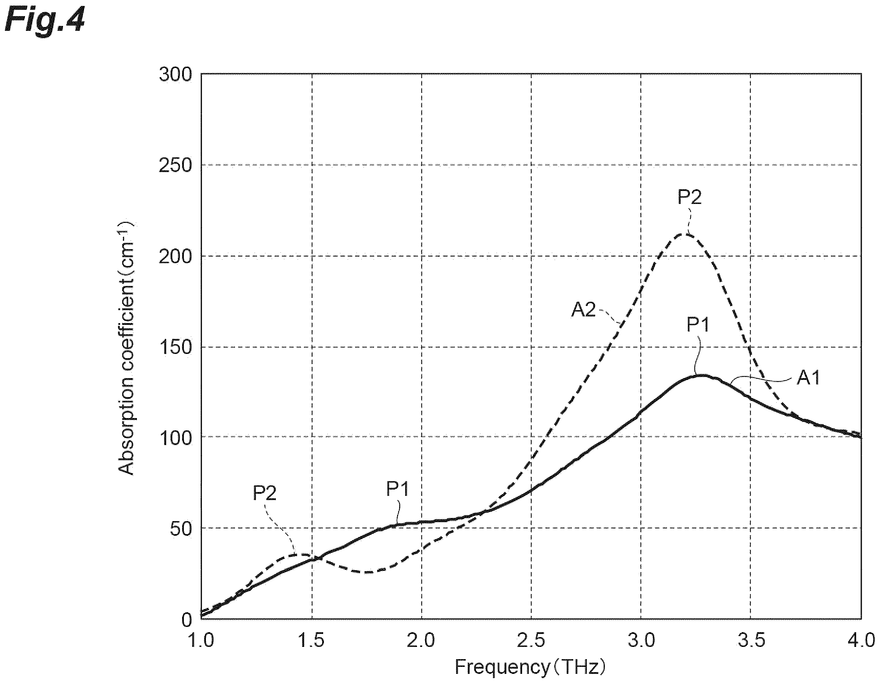

As illustrated in FIG. 4, the frequency characteristic A1 of the anhydride has a first peak P1 at a first frequency. The range of the first frequency is 3.25 THz to 3.45 THz and 1.7 THz to 1.9 THz. That is, the frequency characteristic A1 has a first peak P1 in each of 3.25 THz to 3.45 THz and 1.7 THz to 1.9 THz.

The frequency characteristic A2 of the hydrate has a second peak P2 at a second frequency. The range of the second frequency is 3.1 THz to 3.25 THz and 1.3 THz to 1.5 THz. That is, the frequency characteristic A2 has a second peak P2 in each of 3.1 THz to 3.25 THz and 1.3 THz to 1.5 THz.

The peak of the frequency characteristic refers to a portion of the frequency characteristic in which a change rate of the optical characteristic changes according to a change in frequency. As an example, in a case where the horizontal axis represents a frequency and the vertical axis represents an optical characteristic, when a point indicating an optical characteristic corresponding to a predetermined frequency between one frequency and another frequency is located on one side or the other side with respect to a straight line connecting one point indicating one optical characteristic corresponding to one frequency and another point indicating another optical characteristic corresponding to another frequency, the frequency characteristic has a peak between the one frequency and the another frequency. As another example, in a case where the horizontal axis represents a frequency and the vertical axis represents an optical characteristic, when there is a portion of the frequency characteristic in which the change rate of the optical characteristic changes from a positive number to a negative number or a portion in which the change rate of the optical characteristic changes from a negative number to a positive number according to a change in frequency, the relevant portion is a peak of the frequency characteristic.

Subsequently, the measurement target object S is prepared (step S2). Specifically, the anhydride is arranged in the through hole 32d of the frame body 32 disposed on the reflection surface 30c. Then, water is added to the through hole 32d, and the anhydride and water are mixed to prepare a suspended measurement target object S. In step S2, the suspended measurement target object S prepared in advance may be arranged in the through hole 32d. Step S2 corresponds to a first process.

Subsequently, a substantially constant pressure is applied to the measurement target object S. Specifically, first, the supporting portion 11 is fixed to the frame body 32. Subsequently, the contact portion 12 is arranged on the inner side of the tubular body of the supporting portion 11. Then, the biasing portion 13 is arranged on the inner side of the main body part 121 of the contact portion 12. Accordingly, a substantially constant pressure is applied to the measurement target object S.

Subsequently, the terahertz wave T is entered to the measurement target object S, and the terahertz wave T from the measurement target object S is detected (step S3). As a result, an electric field waveform of the measurement target object S is acquired. In step S3, the terahertz wave T is continuously entered to the reflection surface 30c, and the terahertz wave T from the measurement target object S is continuously detected. As a result, a plurality of electric field waveforms corresponding to a plurality of times separated from each other are acquired. Specifically, in step S3, the adjustment unit 40 sweeps the incident timing of the probe light P2 to the detector 61 over a plurality of times to acquire a plurality of electric field waveforms. Step S3 corresponds to a second process.

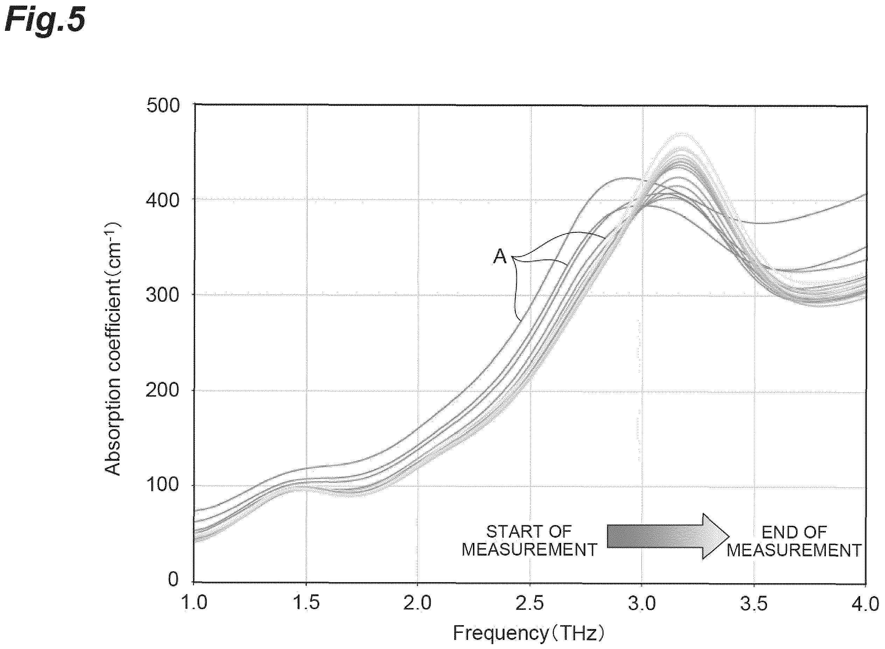

Subsequently, a plurality of frequency characteristics of the measurement target object S are acquired (step S4). Specifically, each frequency characteristic of the measurement target object S is calculated based on the reference electric field waveform and each electric field waveform of the measurement target object S. As illustrated in FIG. 5, in step S4, a plurality of frequency characteristics A are acquired from the start of the measurement to the end of the measurement.

Subsequently, the type of crystal form contained in the measurement target object S is determined based on each frequency characteristic A calculated from each electric field waveform of the measurement target object S. Specifically, whether or not a frequency characteristic A having a first peak at a first frequency exists among the plurality of frequency characteristics A is determined (step S5). If YES in step S5, it is determined that the anhydride is contained in the measurement target object S (step S6). If NO in step S5, it is determined that the anhydride is not contained in the measurement target object S (step S7).

Specifically, whether or not a frequency characteristic A having a second peak at a second frequency exists among the plurality of frequency characteristics A is determined (step S8). If YES in step S8, it is determined that the hydrate is contained in the measurement target object S (step S9). If NO in step S8, it is determined that the hydrate is not contained in the measurement target object S (step S10).

Subsequently, whether or not a frequency characteristic A having a third peak at a third frequency different from the first frequency and the second frequency exists among the plurality of frequency characteristics A is determined (step S11). If YES in step S11, it is determined that the crystal form (hereinafter, referred to as "intermediate") different from the anhydride and the hydrate is contained in the measurement target object S (step S12). If NO in step S11, it is determined that the intermediate is not contained in the measurement target object S (step S13).

Step S5 to step S13 correspond to a third process. As described above, in the third process, in the case when there exists a frequency characteristic having the third peak at the third frequency different from the first frequency related to the first peak corresponding to the anhydride and the second frequency related to the second peak corresponding to the hydrate, it is determined that the crystal form (intermediate) different from the anhydride and the hydrate is contained in the measurement target object S.

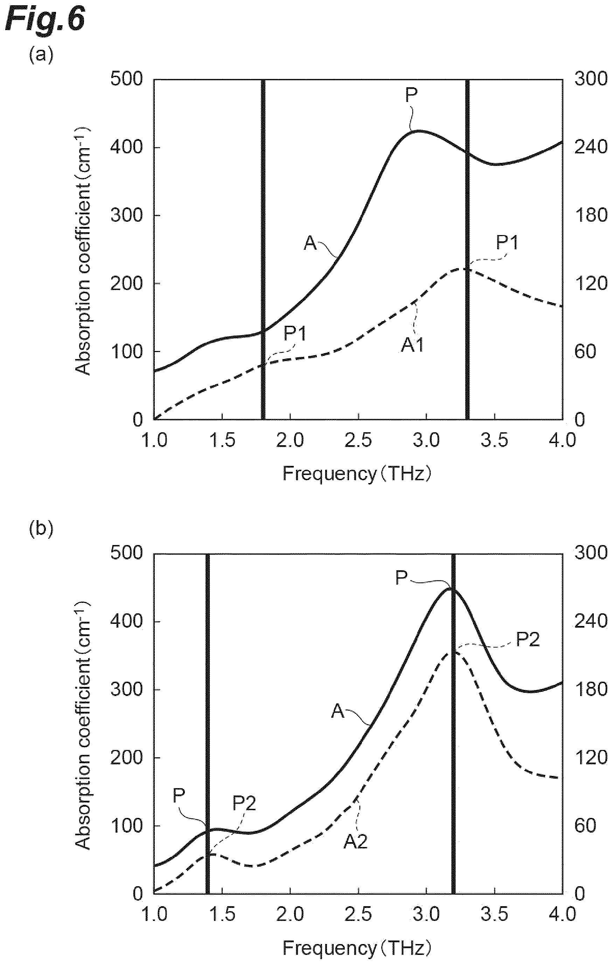

(a) in FIG. 6 is a graph in which the frequency characteristic A1 of the anhydride and the frequency characteristic A of the measurement target object S at the start of the measurement are superimposed. As illustrated in (a) in FIG. 6, the frequency related to the peak P of the frequency characteristic A of the measurement target object S at the start of the measurement is different from the first frequency related to the first peak P1. (b) in FIG. 6 is a graph in which the frequency characteristic A2 of the hydrate and the frequency characteristic A of the measurement target object S at the end of the measurement are superimposed. As illustrated in (b) in FIG. 6, the frequency related to the peak P of the frequency characteristic A of the measurement target object S at the end of the measurement substantially coincides with the second frequency related to the second peak P2.

Referring to (a) and (b) in FIG. 6, it can be seen that the anhydride contained in the measurement target object S is almost completely transferred to the hydrate at the end of the measurement. In addition, since the frequency characteristic A of the measurement target object S at the start of the measurement has a peak (third peak) P at a frequency (third frequency) different from the first frequency and the second frequency, it can be seen that the measurement target object S at the start of the measurement contained a crystal form (intermediate) different from the anhydride and the hydrate. That is, it can be seen that the hydration transfer reaction proceeded from the stage of preparation of the measurement target object S, and the intermediate appeared at the start of the measurement.

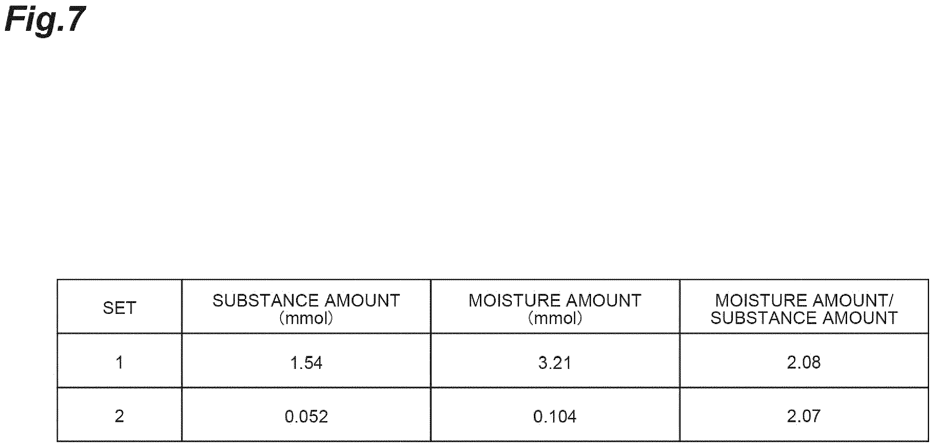

FIG. 7 is a table illustrating the amount of substance contained in the hydrate, the amount of moisture contained in the hydrate, and the value obtained by dividing the amount of moisture by the amount of substance. Each of the substance amount and the moisture amount is a value calculated based on the first weight of the hydrate before the transfer and the second weight of the anhydride after the transfer after the hydrate is transferred to the anhydride by heating the hydrate at the end of the measurement. Specifically, the substance amount is a value calculated based on the second weight. The moisture amount is a value calculated as the moisture amount desorbed from the hydrate based on the difference between the first weight and the second weight. Two sets of such calculations were performed under conditions where the weight of the hydrate is significantly different. As illustrated in FIG. 7, in any set, the value obtained by dividing the moisture amount by the substance amount was approximately 2. With reference to this result, it can be seen that the hydrate at the end of the measurement is a dihydrate.

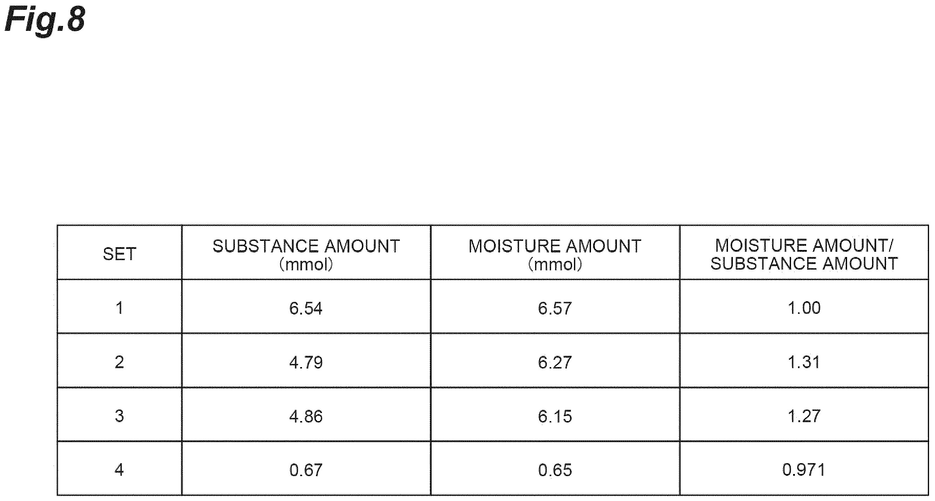

FIG. 8 is a table illustrating the amount of substance contained in the intermediate, the amount of moisture contained in the intermediate, and the value obtained by dividing the amount of moisture by the amount of substance. Each of the substance amount and the moisture amount is a value calculated based on the third weight of the anhydride before the transfer and the fourth weight of the intermediate after the transfer after the anhydride is transferred to the intermediate by humidifying the anhydride in a high-humidity environment. Specifically, the substance amount is a value calculated based on the third weight. The moisture amount is a value calculated as the moisture amount adsorbed on the anhydride based on the difference between the third weight and the fourth weight. Four sets of such calculations were performed under conditions where the weight of the intermediate is significantly different. As illustrated in FIG. 8, in any set, the value obtained by dividing the moisture amount by the substance amount was approximately 1. With reference to this result, it is assumed that the intermediate may be a monohydrate. That is, it is assumed that the intermediate has a possibility of being a crystal form with a moisture amount between the anhydride and the dihydrate.

As described above, in the hydration transfer reaction of phloroglucinol, it was determined that an intermediate, which is a crystal form different from the anhydride and the hydrate, appears. That is, it was determined that an intermediate exists for phloroglucinol.

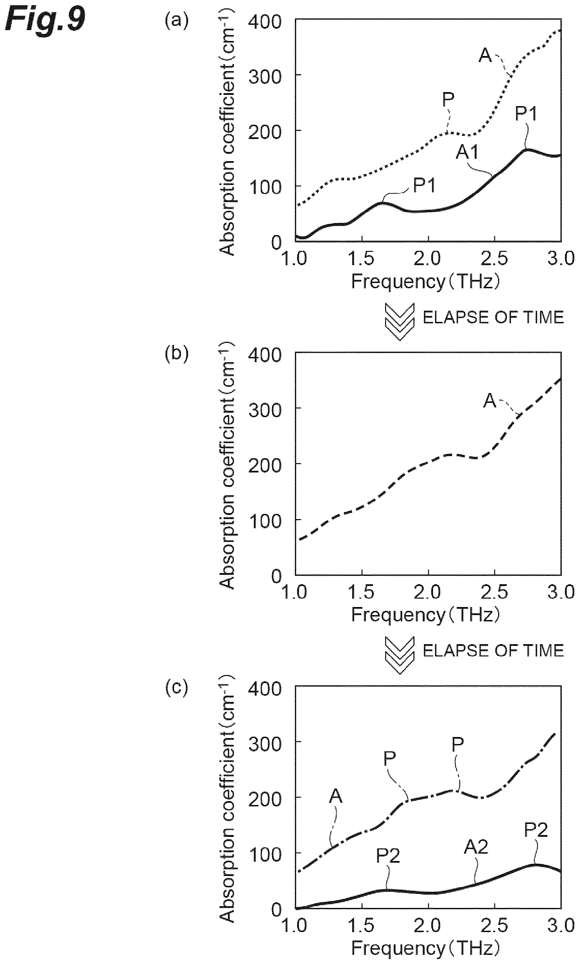

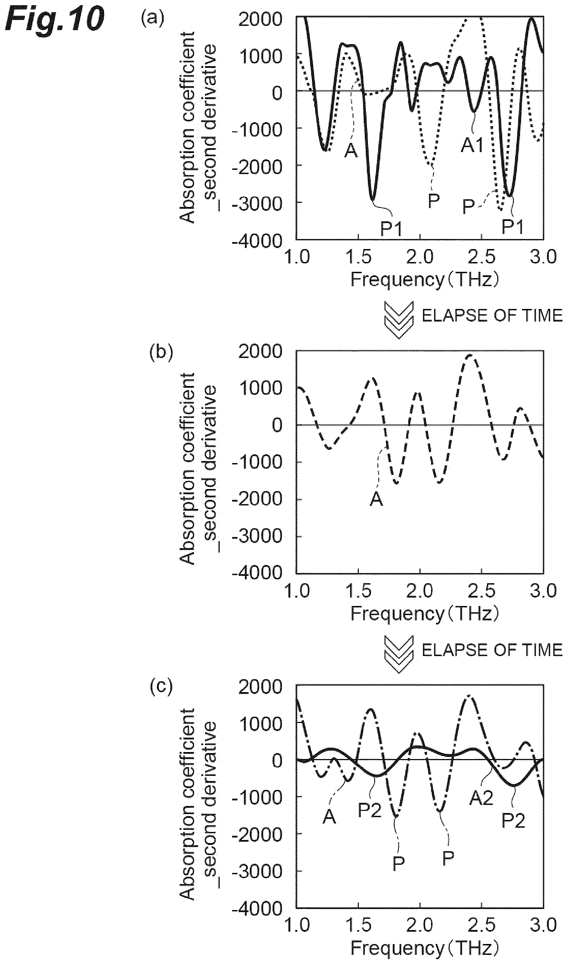

Next, the measurement target object S containing aminophyllin will be described. FIGS. 9 and 10 are diagrams illustrating frequency characteristics of the measurement target object S containing aminophyllin. (a) in FIG. 9 is a graph in which the frequency characteristic A1 of an anhydride of aminophylline (hereinafter, referred to as "anhydride") and the frequency characteristic A of the measurement target object S at the start of the measurement are superimposed. (b) in FIG. 9 is a graph illustrating frequency characteristic A of measurement target object S in the middle of the measurement. (c) in FIG. 9 is a graph in which the frequency characteristic A2 of a hydrate of aminophylline (hereinafter, referred to as "hydrate") and the frequency characteristic A of the measurement target object S at the end of the measurement are superimposed. (a) to (c) in FIG. 10 are diagrams illustrating second derivatives of (a) to (c) in FIG. 9. In the present embodiment, the anhydride of aminophylline is an organic compound of theophylline and ethylenediamine. In this embodiment, the hydrate of aminophylline is a monohydrate of theophylline.

As illustrated in (a) in FIG. 9 and (a) in FIG. 10, the frequency characteristic A1 of the anhydride has a first peak P1 at the first frequency. The first frequency is, for example, about 1.6 THz or about 2.7 THz. That is, the frequency characteristic A1 has a first peak P1 at each of 1.6 THz and 2.7 THz. The frequency related to the peak P of the frequency characteristic A of the measurement target object S at the start of the measurement is different from the first frequency related to the first peak P1. The frequency related to the peak P of the frequency characteristic A of the measurement target object S at the start of the measurement is also different from the second frequency related to the second peak P2 of the frequency characteristic A2 of the hydrate (see (c) in FIG. 9 and (c) in FIG. 10).

Referring to (a) in FIG. 9 and (a) in FIG. 10, since the frequency characteristic A of the measurement target object S at the start of the measurement has a peak (third peak) P at a frequency (third frequency) different from the first frequency and the second frequency, it can be seen that the measurement target object S at the start of the measurement contained a crystal form (first intermediate) different from the anhydride and the hydrate. That is, it can be seen that the hydration transfer reaction proceeded from the stage of preparation of the measurement target object S, and the first intermediate appeared at the start of the measurement.

As illustrated in (c) in FIG. 9 and (c) in FIG. 10, the frequency characteristic A2 of the hydrate has a second peak P2 at the second frequency. The second frequency is, for example, about 1.7 THz or about 2.8 THz. That is, the frequency characteristic A2 has a second peak P2 at each of 1.7 THz and 2.8 THz. The frequency related to the peak P of the frequency characteristic A of the measurement target object S at the end of the measurement is different from the second frequency related to the second peak P2. The frequency related to the peak P of the frequency characteristic A of the measurement target object S at the end of the measurement is different from the first frequency related to the first peak P1 of the frequency characteristic A1 of the anhydride and the frequency related to the peak P of the frequency characteristic A of the measurement target object S at the start of the measurement (see (a) in FIG. 9 and (a) in FIG. 10).

Referring to (c) in FIG. 9 and (c) in FIG. 10, since the frequency characteristic A of the measurement target object S at the end of the measurement has a peak (third peak) P at a frequency (third frequency) different from the first frequency and the second frequency, it can be seen that the measurement target object S at the end of the measurement contained a crystal form (second intermediate) different from the anhydride and the hydrate. That is, it can be seen that a second intermediate different from the first intermediate appeared at the end of the measurement. This is assumed to be because at the start of the measurement, the anhydride has transferred to the first intermediate, and after a predetermined period has elapsed, the first intermediate may have transferred to the second intermediate.

As described above, in the hydration transfer reaction of aminophylline, it was determined that an intermediate which is a crystal form different from the anhydride and the hydrate appears. That is, it was determined that an intermediate exists for aminophylline.

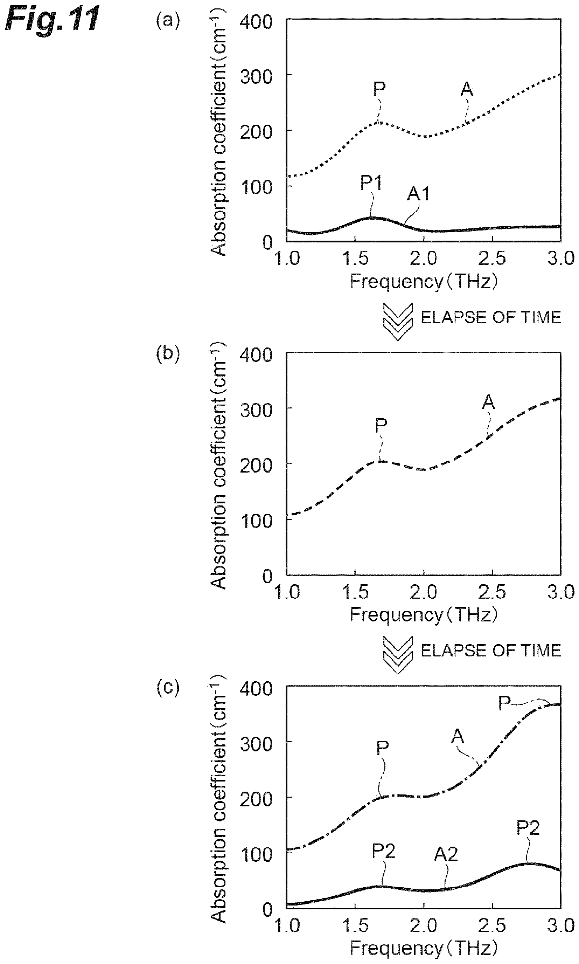

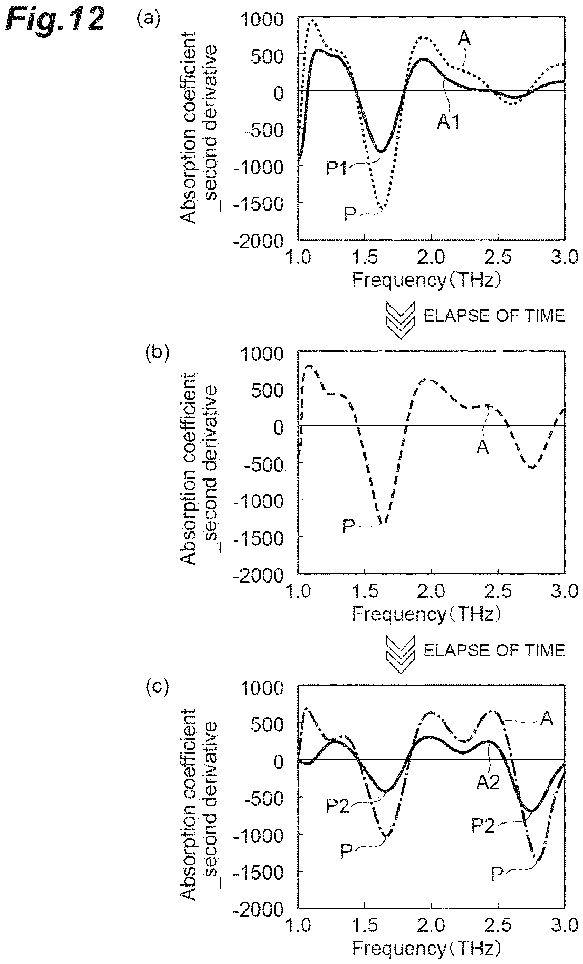

Next, the measurement target object S containing theophylline will be described. FIGS. 11 and 12 are diagrams illustrating frequency characteristics of the measurement target object S containing theophylline. (a) in FIG. 11 is a graph in which the frequency characteristic A1 of an anhydride of theophylline (hereinafter, referred to as "anhydride") and the frequency characteristic A of the measurement target object S at the start of the measurement are superimposed. (b) in FIG. 11 is a graph illustrating frequency characteristic A of measurement target object S in the middle of the measurement. (c) in FIG. 11 is a graph in which the frequency characteristic A2 of a hydrate of theophylline (hereinafter, referred to as "hydrate") and the frequency characteristic A of the measurement target object S at the end of the measurement are superimposed. (a) to (c) in FIG. 12 are diagrams illustrating second derivatives of (a) to (c) in FIG. 11.

As illustrated in (a) in FIG. 11 and (a) in FIG. 12, the frequency characteristic A1 of the anhydride has a first peak P1 at the first frequency. The first frequency is, for example, about 1.6 THz. That is, the frequency characteristic A1 has a first peak P1 at 1.6 THz. The frequency related to the peak P of the frequency characteristic A of the measurement target object S at the start of the measurement substantially coincides with the first frequency related to the first peak P1.

As illustrated in (c) in FIG. 11 and (c) in FIG. 12, the frequency characteristic A2 of the hydrate has a second peak P2 at the second frequency. The second frequency is, for example, about 1.7 THz or about 2.8 THz. That is, the frequency characteristic A2 has a second peak P2 at each of 1.7 THz and 2.8 THz. The frequency related to the peak P of the frequency characteristic A of the measurement target object S at the end of the measurement substantially coincides with the second frequency related to the second peak P2.

As illustrated in (b) in FIG. 11 and (b) in FIG. 12, the frequency characteristic A in the middle of measurement does not have a peak at a frequency different from the first frequency and the second frequency.

Referring to FIGS. 11 and 12, since the frequency characteristic A having a peak at a frequency different from the first frequency and the second frequency does not exist among the plurality of frequency characteristics A, it can be seen that the measurement target object S did not contain a crystal form (intermediate) different from the anhydride and the hydrate.

As described above, in the hydration transfer reaction of theophylline, it was determined that an intermediate which is a crystal form different from the anhydride and the hydrate did not appear. That is, it was determined that an intermediate does not exist for theophylline.

As described above, in the crystal form determination method according to the first embodiment, in the case when a frequency characteristic A having the third peak at the third frequency different from the first frequency and the second frequency exists among the plurality of frequency characteristics A, it is determined that the crystal form (intermediate) different from the anhydride and the hydrate is contained in the measurement target object S. As a result, whether or not the crystal form different from the anhydride and the hydrate exists can be determined for the measurement target object S, and eventually, various objects-to-be-measured S can be classified into the measurement target object S in which the crystal form different from the anhydride and the hydrate exists and the measurement target object S in which the crystal form different from the anhydride and the hydrate does not exist. Therefore, according to the crystal form determination method, detailed information on the measurement target object S can be acquired.

If determined in step S5 that a frequency characteristic A having a first peak at the first frequency exists among the plurality of frequency characteristics A, it is determined in step S6 that the anhydride is contained in the measurement target object S. This makes it possible to determine not only the crystal form different from the anhydride and the hydrate but also whether or not the anhydride is contained in the measurement target object S. Therefore, more detailed information about the measurement target object S can be acquired.

If determined in step S8 that a frequency characteristic having a second peak at the second frequency exists among the plurality of frequency characteristics A, it is determined in step S9 that the hydrate is contained in the measurement target object S. This makes it possible to determine not only the crystal form different from the anhydride and the hydrate but also whether or not the hydrate is contained in the measurement target object S. Therefore, more detailed information about the measurement target object S can be acquired.

[Second Embodiment]

Next, a crystal form determination method according to a second embodiment using the spectroscopic device 1 will be described. In the present embodiment, the type of crystal form was determined for the measurement target object S containing phloroglucinol.

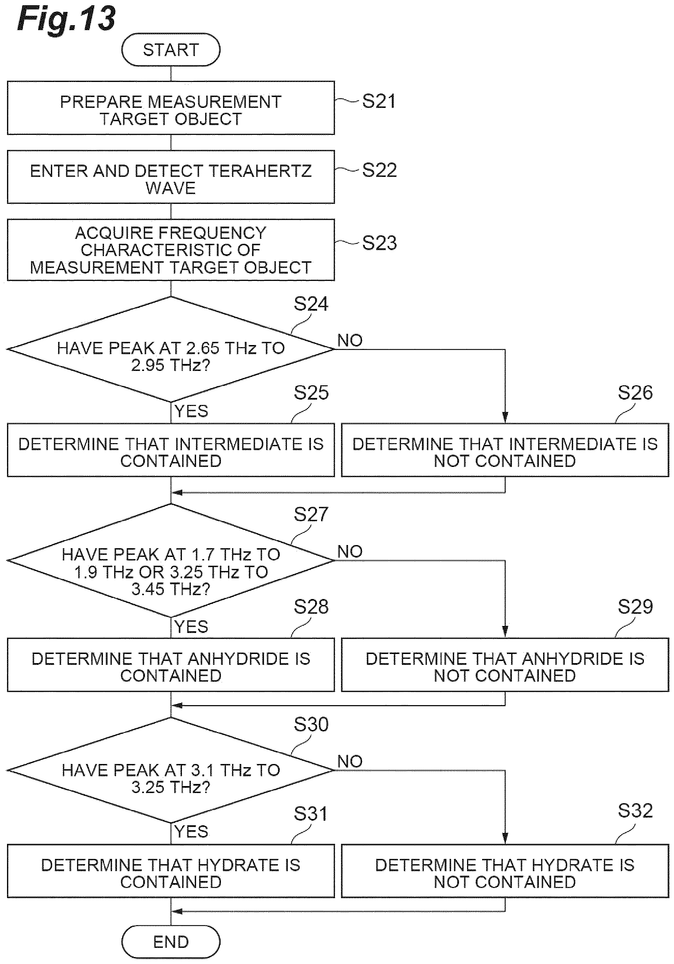

As illustrated in FIG. 13, first, a measurement target object S containing phloroglucinol is prepared (step S21). Specifically, the measurement target object S is arranged in the through hole 32d of the frame body 32 disposed on the reflection surface 30c. Step S21 corresponds to a first process. Subsequently, a substantially constant pressure is applied to the measurement target object S, as in the first embodiment.

Subsequently, the terahertz wave T is entered to the measurement target object S, and the terahertz wave T from the measurement target object S is detected (step S22). As a result, an electric field waveform of the measurement target object S is acquired. In step S22, for example, one electric field waveform is acquired. In step S22, the terahertz wave T of a broadband wavelength is entered to the measurement target object S. The terahertz wave T has a frequency in a range of at least 2.65 THz to 2.95 THz. In the present embodiment, the terahertz wave T has a frequency in a range of 1 THz to 4 THz. Step S22 corresponds to a second process.

Subsequently, a plurality of frequency characteristics of the measurement target object S are acquired (step S23). Specifically, the frequency characteristic of the measurement target object S is calculated based on the reference electric field waveform and the electric field waveform of the measurement target object S.

Subsequently, the type of crystal form contained in the measurement target object S is determined based on each frequency characteristic calculated from the electric field waveform of the measurement target object S. Specifically, whether or not the frequency characteristic has a peak in 2.65 THz to 2.95 THz is determined (step S24). If YES in step S24, it is determined that the crystal form (intermediate) different from the anhydride and the hydrate is contained in the measurement target object S (step S25). If NO in step S24, it is determined that the intermediate is not contained in the measurement target object S (step S26).

Subsequently, whether or not the frequency characteristic has a peak in 1.7 THz to 1.9 THz or 3.25 THz to 3.45 THz is determined (step S27). If YES in step S27, it is determined that the anhydride is contained in the measurement target object S (step S28). If NO in step S27, it is determined that the anhydride is not contained in the measurement target object S (step S29).

Subsequently, whether or not the frequency characteristic has a peak in 3.1 THz to 3.25 THz is determined (step S30). If YES in step S30, it is determined that the hydrate is contained in the measurement target object S (step S31). If NO in step S30, it is determined that the hydrate is not contained in the measurement target object S (step S32).

Step S24 to step S32 correspond to a third process. As described above, in the third process, when the frequency characteristic of the measurement target object S has a peak in 2.65 THz to 2.95 THz, it is determined that a crystal form different from the anhydride and the hydrate is contained in the measurement target object S.

In the third process, when the frequency characteristic of the measurement target object S has a peak in 1.3 THz to 1.5 THz and does not have a peak in 3.1 THz to 3.25 THz, it may be determined that the intermediate is contained in the measurement target object S. In the third process, when the frequency characteristic of the measurement target object S has a peak in 1.3 THz to 1.5 THz and has a peak in 3.1 THz to 3.25 THz, it may be determined that the hydrate is contained in the measurement target object S.

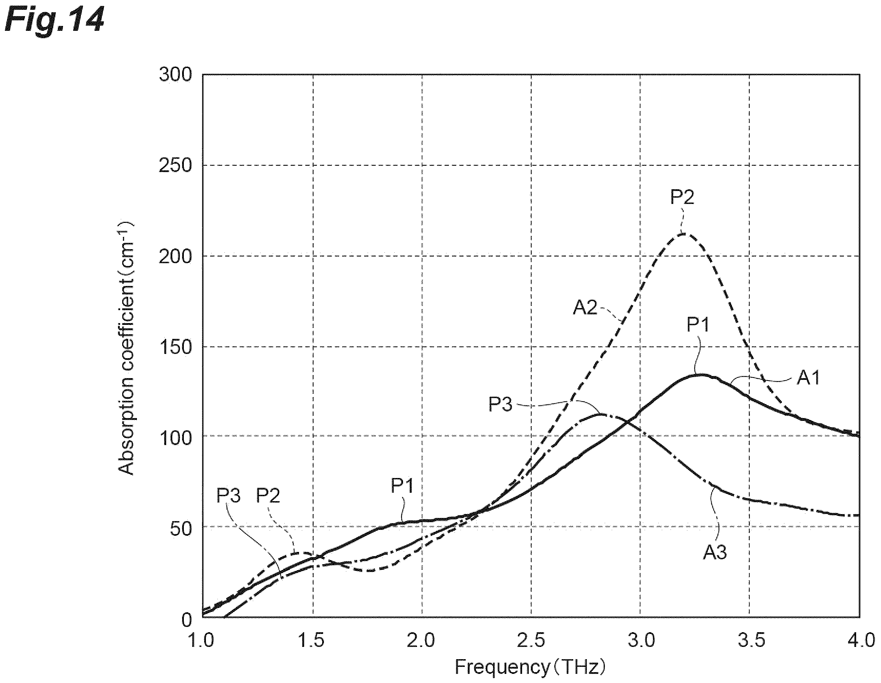

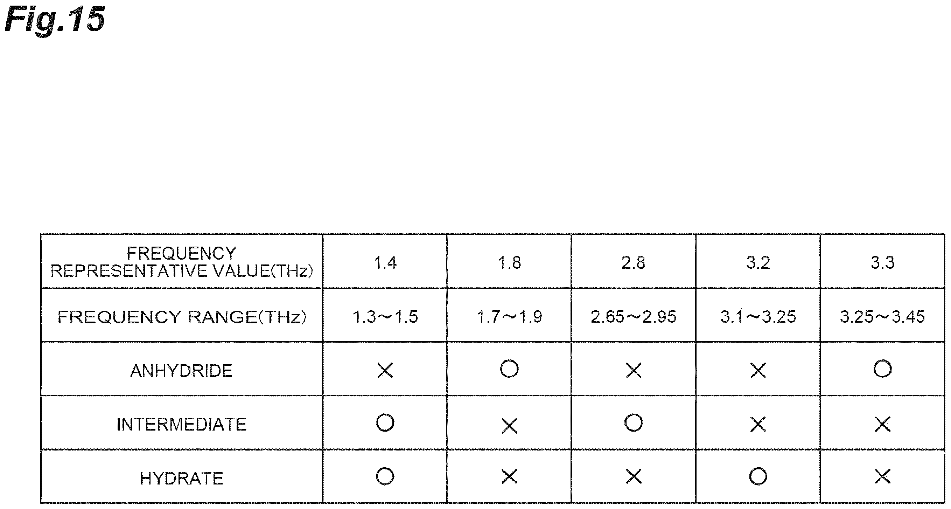

FIG. 14 is a diagram illustrating frequency characteristics A1 of an anhydride of phloroglucinol (hereinafter, referred to as "anhydride"), frequency characteristics A2 of a hydrate of phloroglucinol (hereinafter, referred to as "hydrate"), and frequency characteristics A3 of an intermediate of phloroglucinol (hereinafter, referred to as "intermediate"). FIG. 15 is a table illustrating frequencies corresponding to peaks of the frequency characteristics A1, A2, and A3. In the present embodiment, the hydrate of phloroglucinol is a dihydrate.

As illustrated in FIGS. 14 and 15, the frequency characteristic A1 of the anhydride has a first peak P1 at a first frequency. The range of the first frequency is 3.25 THz to 3.45 THz and 1.7 THz to 1.9 THz. That is, the frequency characteristic A1 has the first peak P1 at each of 3.25 THz to 3.45 THz (the representative value is 3.3 THz) and 1.7 THz to 1.9 THz (the representative value is 1.8 THz).

The frequency characteristic A2 of the hydrate has a second peak P2 at a second frequency. The range of the second frequency is 3.1 THz to 3.25 THz (the representative value is 3.2 THz) and 1.3 THz to 1.5 (the representative value is 1.4 THz) THz. That is, the frequency characteristic A2 has a second peak P2 in each of 3.1 THz to 3.25 THz and 1.3 THz to 1.5 THz.

The frequency characteristic A3 of the intermediate has a third peak P3 at the third frequency. The range of the third frequency is 2.65 THz to 2.95 THz (the representative value is 2.8 THz) and 1.3 THz to 1.5 THz (the representative value is 1.4 THz). That is, the frequency characteristic A3 has a third peak P3 at each of 2.65 THz to 2.95 THz and 1.3 THz to 1.5 THz. Such frequency characteristics A1, A2, and A3 may be reference data stored in advance, or may be calculated based on the electric field waveform and the reference electric field waveform acquired for each of the anhydride, the hydrate, and the intermediate before step S21.

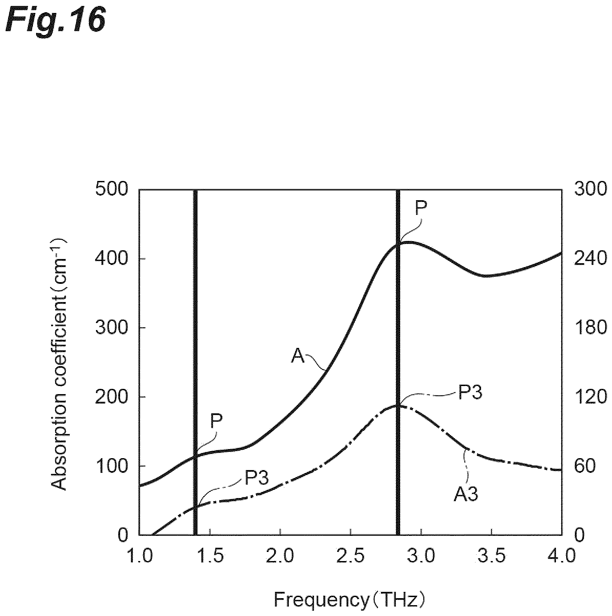

FIG. 16 is a graph in which the frequency characteristic A3 of the intermediate and the frequency characteristic A of the measurement target object S are superimposed. As illustrated in FIG. 16, the frequency related to the peak P of the frequency characteristic A of the measurement target object S substantially coincides with the third frequency related to the third peak P3. Referring to FIG. 16, it can be seen that the measurement target object S contains a crystal form (intermediate) different from the anhydride and the hydrate.

As described above, in the crystal form determination method according to the second embodiment, in the case when the frequency characteristic A has a peak in 2.65 THz to 2.95 THz, it is determined that a crystal form (intermediate) different from an anhydride and a hydrate is contained in the measurement target object S. This makes it possible to determine whether or not the measurement target object S containing phloroglucinol contains a crystal form different from the anhydride and the hydrate. Therefore, according to the crystal form determination method, detailed information on the measurement target object S can be acquired.

In step S27, when the frequency characteristic has a peak in 1.7 THz to 1.9 THz or 3.25 THz to 3.45 THz, it is determined that the anhydride is contained in the measurement target object S. This makes it possible to determine not only the crystal form different from the anhydride and the hydrate but also whether or not the anhydride is contained in the measurement target object S. Therefore, more detailed information about the measurement target object S can be acquired.

In step S30, when the frequency characteristic has a peak in 3.1 THz to 3.25 THz, it is determined that the hydrate is contained in the measurement target object S. This makes it possible to determine not only the crystal form different from the anhydride and the hydrate but also whether or not the hydrate is contained in the measurement target object S. Therefore, more detailed information about the measurement target object S can be acquired.

In step S22, the terahertz wave T of a broadband wavelength having a frequency in a range of at least 2.65 THz to 2.95 THz is entered to the measurement target object S. As a result, the terahertz wave T of a broadband wavelength can be collectively entered to the measurement target object S, and information on the measurement target object S can be efficiently acquired.

According to this crystal form determination method, for example, the physicochemical characteristic of a medicine can be accurately evaluated. Specifically, when the crystal form of the medicine changes, the physicochemical characteristic such as solubility or dissolution rate of the medicine in water changes, and as a result, the drug efficacy also changes. Therefore, accurate evaluation of the crystal form is very important. According to this crystal form determination method, an intermediate different from an anhydride and a hydrate can also be evaluated, so that the crystal form of a medicine can be more accurately evaluated. As a result, it is possible to improve the ability to elucidate the physicochemical characteristics of a medicine and to realize more secure and safe development or manufacturing of a medicine.

This crystal form determination method can be applied not only to the characteristic evaluation at the time of medicine development as described above, but also to, for example, quality inspection at the time of carrying-in or storing raw medicine, or quality evaluation at the time of manufacturing or after manufacturing. According to this crystal form determination method, as an example, not only the state of transfer to a hydrate but also the state of transfer to an intermediate can be grasped for a medicine having a drug efficacy only as an anhydride, so that the possibility of transfer of the anhydride during transportation or during storage can be grasped more accurately.

In addition, according to this crystal form determination method, for example, the amount of substance can be accurately managed. Accurate evaluation of the amount of substance at the time of synthesis or the like is extremely important. For example, when no intermediate is contained, the weight of the net phloroglucinol contained in the 1 t of phloroglucinol anhydride is about 1 t, whereas the weight of the net phloroglucinol contained in the 1 t of phloroglucinol dihydrate is about 777.7 kg. When no intermediate is contained, the weight of the net phloroglucinol can be accurately grasped based on this relationship. In other words, when an intermediate is contained, it becomes difficult to grasp the weight of the net phloroglucinol. Therefore, for example, if the presence of the intermediate and the hydration amount contained in the intermediate can be evaluated, the amount of phloroglucinol used in the reaction can be more accurately evaluated or calculated. According to the accurate determination of the crystal form, the weight or substance amount of the reactant in the industrial process or the chemical reaction process can be accurately weighed, and higher quality product production can be performed. This crystal form determination method can also be applied to sample measurement before chemical synthesis.

[Modified Example]

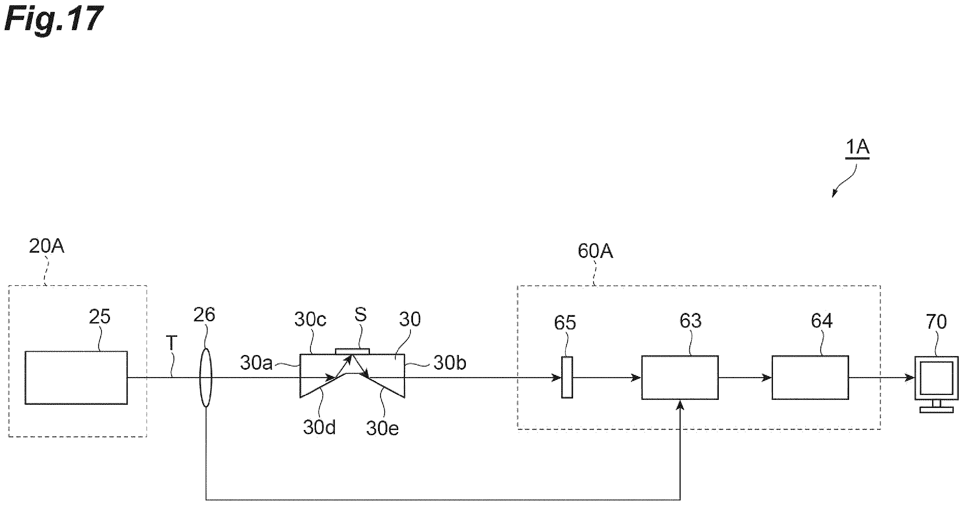

The present disclosure is not limited to the above embodiments. In the crystal form determination method according to the second embodiment, an example in which the spectroscopic device 1 is used has been shown, but as shown in FIG. 17, a spectroscopic device 1A may be used in the crystal form determination method according to the second embodiment. The spectroscopic device 1A includes an output unit 20A instead of the output unit 20, and includes a detection unit 60A instead of the detection unit 60. The spectroscopic device 1A does not include the adjustment unit 40 and the reflection unit 50. The spectroscopic device 1A includes an output unit 20A, a chopper 26, an arrangement unit 30, a detection unit 60A, and a processing unit 70.

The output unit 20A includes a plurality of light sources 25. Each light source 25 outputs a terahertz wave T having a single wavelength. Each light source 25 outputs a terahertz wave T having frequencies different from each other. The light source 25 is, for example, a backward wave tube or a quantum cascade laser. The chopper 26 alternately repeats passing and shielding of the terahertz wave T output from the light source 25 at a constant cycle. The terahertz wave T output from the output unit 20A is entered to the incident surface 30a of the arrangement unit 30, sequentially reflected by the first sub-reflection surface 30d, the reflection surface 30c, and the second sub-reflection surface 30e, then output from the exit surface 30b to the outside, and entered to the detection unit 60A.

The detection unit 60A detects the terahertz wave T output from the arrangement unit 30. Specifically, the detection unit 60A includes a detector 65, a lock-in amplifier 63, and an A/D converter 64. The detector 65 is, for example, a Golay cell, a bolometer, a Schottky barrier diode, a resonant tunnel diode, or the like. The electrical signal output from the detector 65 is input to the lock-in amplifier 63. The lock-in amplifier 63 synchronously detects the electrical signal output from the detector 65 at a repetition frequency of passing and shielding of the terahertz wave T in the chopper 23. The A/D converter 64 converts the analog signal from the lock-in amplifier 63 into a digital signal. The processing unit 70 calculates frequency characteristics based on the signal output from the A/D converter 64. Note that the spectroscopic device 1A may not include the chopper 26 and the lock-in amplifier 63.

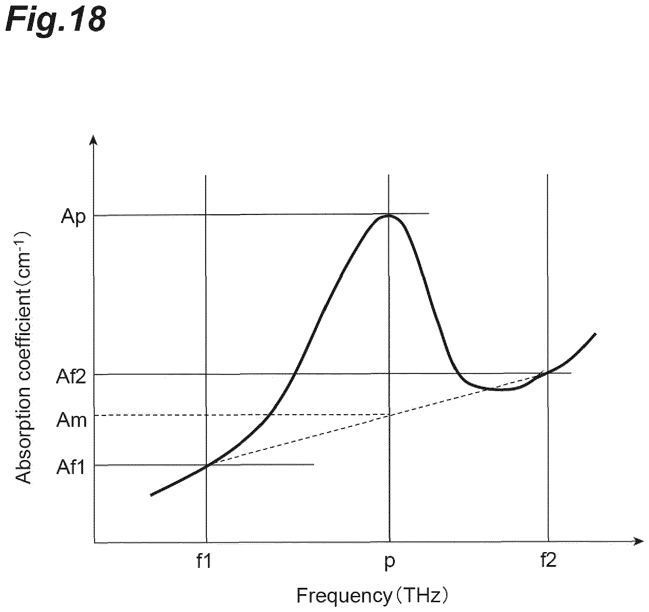

Next, a crystal form determination method using the spectroscopic device 1A will be described. In this crystal form determination method, the terahertz wave T having a single wavelength is entered to the measurement target object S. The terahertz wave T has at least one frequency selected from a range of at least 2.65 THz to 2.95 THz. In the present embodiment, the terahertz wave T has at least one frequency selected from a range of 1 THz to 4 THz. Specifically, as illustrated in FIG. 18, each of the terahertz wave T having a frequency of p, the terahertz wave T having a frequency of f1, and the terahertz wave T having a frequency of f2 is entered to the measurement target object S (step S22). p is one value selected from the range of 2.65 THz to 2.95 THz. f1 is a value smaller than p. f2 is a value larger than p.

Subsequently, Ap, Af1, and Af2 are calculated as frequency characteristics in a case where the frequencies are p, f1, and f2. Ap, Af1, and Af2 are, for example, absorbance. Subsequently, Am is calculated based on

$$Am = (Af2 - Af1) \times (p - f1)/(f2 - f1) + Af1 \text{ (Formula 1).}$$

Subsequently, whether or not the intermediate is contained in the measurement target object S is determined based on Ap > Am + α (Formula 2). When Formula 2 is satisfied, it is determined that the intermediate is contained in the measurement target object S, and when Formula 2 is not satisfied, it is determined that the intermediate is not contained in the measurement target object S. Note that α is a buffer. α can be appropriately set according to the situation of the spectroscopic device 1A. α can be set based on the noise or the like at the time of measurement by the spectroscopic device 1A. As an example, α is a value three times the standard deviation of the measurement variation.

As frequency characteristics when the frequency is p, f1, and f2, transmitted light intensities Ip, If1, and If2 may be used instead of the absorbances Ap, Af1, and Af2. In that case, Im is calculated based on Im = (If2 - If1) × (p - f1)/(f2 - fl) + If1 (Formula 3). Subsequently, whether or not the intermediate is contained in the measurement target object S is determined based on Ip < Im - α (Formula 4). When Formula 4 is satisfied, it is determined that the intermediate is contained in the measurement target object S, and when Formula 4 is not satisfied, it is determined that the intermediate is not contained in the measurement target object S. In this case, the reference measurement may not be performed.

Similarly to the intermediate, whether the anhydride or the hydrate is contained in the measurement target object S is determined based on the Formulas 1 and 2 or the Formulas 3 and 4. When determining for the anhydride, p is one value selected from 1.7 THz to 1.9 THz or one value selected from 3.25 THz to 3.45 THz. When determining for the hydrate, p is one value selected from 3.1 THz to 3.25 THz.

According to the crystal form determination method using the spectroscopic device 1A, information on the measurement target object S can be acquired with a simple configuration.

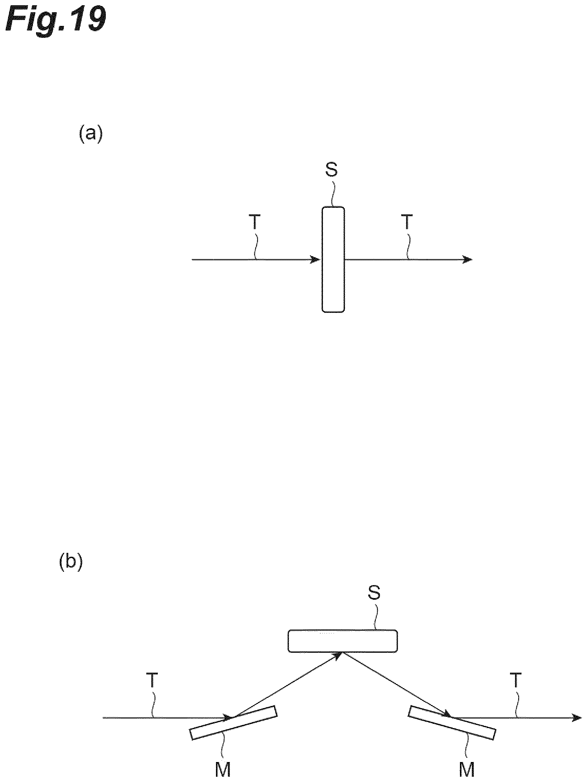

In addition, the spectroscopic devices 1 and 1A may not be devices for implementing the attenuated total reflection spectroscopy. As illustrated in (a) in FIG. 19, in the spectroscopic devices 1 and 1A, the terahertz wave T may be transmitted through the measurement target object S. As illustrated in (b) in FIG. 19, in the spectroscopic devices 1 and 1A, the terahertz wave T may be detected after being reflected by the pair of mirrors M and the measurement target object S.

In the first embodiment, an example in which the anhydride is transferred to the hydrate has been described, but the hydrate may be transferred to the anhydride. That is, the measurement target object S may be accompanied by a dehydration reaction of a hydrate.

Furthermore, in the first embodiment, the temperature of the measurement target object S may be adjusted. Accordingly, by keeping the temperature of the measurement target object S constant, the temporal change of the frequency characteristic of the measurement target object S can be measured under a predetermined temperature condition. Therefore, information on the measurement target object S can be acquired with good reproducibility.

In addition, an optical interference method may be used as the optical system of the detection units 60 and 60A. In this case, the absorption spectrum of the terahertz wave T can be directly acquired without acquiring the electric field waveform of the terahertz wave T by the detection units 60 and 60A.

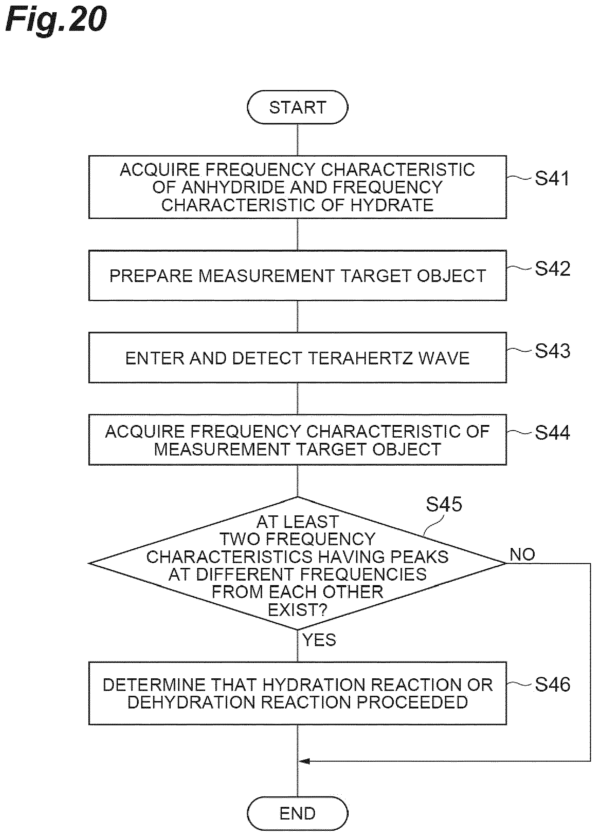

Next, a method for determining whether or not the hydration reaction or the dehydration reaction proceeded in the measurement target object S will be described. In the third process of the first embodiment, whether or not the hydration reaction of the anhydride or the dehydration reaction of the hydrate proceeded in the measurement target object S may be determined. Specifically, as illustrated in FIG. 20, first, as in step S1, the frequency characteristics of the anhydride (e.g., anhydride of phloroglucinol, anhydride of aminophyllin, or anhydride of theophylline) and the frequency characteristics of the hydrate (e.g., hydrate of phloroglucinol, hydrate of aminophyllin, or hydrate of theophylline) are acquired (step S41).

Subsequently, as in step S2, the measurement target object S is prepared (step S42). Subsequently, as in step S3, the terahertz wave T is entered to the measurement target object S, and the terahertz wave T from the measurement target object S is detected (step S43). Then, as in step S4, a plurality of frequency characteristics of the measurement target object S are acquired (step S44). Subsequently, whether or not at least two frequency characteristics having peaks at frequencies different from each other exist among the plurality of frequency characteristics is determined (step S45). The two frequency characteristics may be, for example, a frequency characteristic having a first peak at a first frequency (frequency characteristic corresponding to an anhydride) and a frequency characteristic having a second peak at a second frequency (frequency characteristic corresponding to a hydrate). The two frequency characteristics may be, for example, a frequency characteristic having a first peak at a first frequency and a frequency characteristic having a peak at a frequency different from the first frequency and the second frequency. The two frequency characteristics may be, for example, a frequency characteristic having a second peak at a second frequency and a frequency characteristic having a peak at a frequency different from the first frequency and the second frequency.

When one frequency characteristic has a plurality of peaks, "having peaks at frequencies different from each other" means that one frequency characteristic of the two frequency characteristics has a peak at a frequency different from the frequencies of all peaks of the other frequency characteristic of the two frequency characteristics. In other words, "having peaks at frequencies different from each other" means that frequencies of all peaks of one frequency characteristic of the two frequency characteristics do not coincide with frequencies of all peaks of the other frequency characteristic of the two frequency characteristics. In this manner, in step S45, whether or not the peak position of the frequency characteristic changes with time is determined. If YES in step S45, it is determined that the hydration reaction of the anhydride or the dehydration reaction of the hydrate proceeded in the measurement target object S (step S46). In step S46, it is determined that the anhydride has transferred to the hydrate in the measurement target object S, or it is determined that the hydrate has transferred to the anhydride in the measurement target object S.

If NO in step S45, the determination process is ended. Step S45 to step S46 correspond to a third process. As described above, in the third process, when at least two frequency characteristics having peaks at frequencies different from each other exist among the plurality of frequency characteristics, it is determined that the hydration reaction of the anhydride or the dehydration reaction of the hydrate proceeded in the measurement target object S. This makes it possible to determine whether or not the hydration reaction of the anhydride or the dehydration reaction of the hydrate proceeded in the measurement target object S. When it is determined that the hydration reaction of the anhydride or the dehydration reaction of the hydrate proceeded in the measurement target object S, whether or not a crystal form (intermediate) different from the anhydride and the hydrate exists in the measurement target object S is determined. That is, by determining whether or not at least two frequency characteristics having peaks at frequencies different from each other exist, whether or not the hydration reaction or the dehydration reaction proceeded in the measurement target object S can be determined, and whether or not the intermediate appears according to the proceeding of the hydration reaction or the dehydration reaction can be determined. Therefore, more detailed information about the measurement target object S can be acquired.

In step S45, whether or not the hydration reaction (or dehydration reaction) of the measurement target object proceeded may be determined by determining whether or not the peak intensity (peak magnitude) of the frequency characteristics changes over time (e.g., when one peak and the other peak exist in one frequency characteristic, by determining whether or not the peak intensity of one peak decreases and the peak intensity of the other peak increases).

In the first embodiment, the third process may be performed simultaneously with the second process. Specifically, the type of crystal form contained in the measurement target object S may be determined while entering the terahertz wave T to the measurement target object S and detecting the terahertz wave T from the measurement target object S. Furthermore, in the third process, whether or not the hydration reaction or the dehydration reaction proceeded in the measurement target object S may be determined while entering the terahertz wave T to the measurement target object S and detecting the terahertz wave T from the measurement target object S. In the third process, whether or not a frequency characteristic having the first peak, the second peak, or the third peak exists may be determined every time the frequency characteristic corresponding to the electric field waveform acquired in the second process is acquired. In the third process, when the frequency characteristic having the second peak is acquired after the frequency characteristic having the first peak is acquired, it may be determined that the hydration reaction of the anhydride proceeded in the measurement target object S. In the third process, when the frequency characteristic having the first peak is acquired after the frequency characteristic having the second peak is acquired, it may be determined that the dehydration reaction of the hydrate proceeded in the measurement target object S.

Reference Signs List

A       Frequency characteristic

P1      First peak

P2      Second peak

P3      Third peak

S       Measurement target object

T       Terahertz wave.

**Claims**

1. A crystal form determination method comprising:

   a first process of preparing a measurement target object;
   a second process of entering a terahertz wave with respect to the measurement target object and detecting the terahertz wave from the measurement target object to acquire a plurality of detection results corresponding to a plurality of times separated from each other; and
   a third process of determining a type of crystal form contained in the measurement target object based on a plurality of frequency characteristics calculated from the plurality of detection results; wherein
   in the third process, when a frequency characteristic having a third peak at a third frequency different from a first frequency related to a first peak corresponding to an anhydride and a second frequency related to a second peak corresponding to a hydrate exists among the plurality of frequency characteristics, determination is made that a crystal form different from the anhydride and the hydrate is contained in the measurement target object.

2. The crystal form determination method according to claim 1, wherein in the third process, when a frequency characteristic having the first peak at the first frequency exists among the plurality of frequency characteristics, determination is made that the anhydride is contained in the measurement target object.

3. The crystal form determination method according to claim 1, wherein in the third process, when a frequency characteristic having the second peak at the second frequency exists among the plurality of frequency characteristics, determination is made that the hydrate is contained in the measurement target object.

4. The crystal form determination method according to claim 1, wherein in the third process, when at least two frequency characteristics having peaks at frequencies different from each other exist among the plurality of frequency characteristics, determination is made that a hydration reaction of an anhydride or a dehydration reaction of a hydrate proceeded in the measurement target object.

5. A crystal form determination method comprising:

   a first process of preparing a measurement target object containing phloroglucinol;
   a second process of entering a terahertz wave with respect to the measurement target object and detecting the terahertz wave from the measurement target object to acquire a detection result related to the measurement target object; and
   a third process of determining a type of crystal form contained in the measurement target object based on a frequency characteristic calculated from the detection result; wherein
   in the third process, when a frequency characteristic has a peak in 2.65 THz to 2.95 THz, determination is made that a crystal form different from the anhydride and the hydrate is contained in the measurement target object.

6. The crystal form determination method according to claim 5, wherein in the third process, when the frequency characteristic has a peak in 1.7 THz to 1.9 THz or 3.25 THz to 3.45 THz, determination is made that the anhydride is contained in the measurement target object.

7. The crystal form determination method according to claim 5, wherein in the third process, when the frequency characteristic has a peak in 3.1 THz to 3.25 THz, determination is made that the hydrate is contained in the measurement target object.

8. The crystal form determination method according to claim 5, wherein in the second process, the terahertz wave of a broadband wavelength having a frequency in a range of at least 2.65 THz to 2.95 THz is entered to the measurement target object.

9. The crystal form determination method according to claim 5, wherein in the second process, the terahertz wave of a single wavelength having at least one frequency selected from a range of at least 2.65 THz to 2.95 THz is entered to the measurement target object.

Fig.1

# *Fig.2*

**Fig.3**

```
( START )
       │
┌──────────────────────────────────────┐
│ ACQUIRE FREQUENCY CHARACTERISTIC      │  S1
│ OF ANHYDRIDE AND FREQUENCY            │
│ CHARACTERISTIC OF HYDRATE             │
└──────────────────────────────────────┘
       │
┌──────────────────────────────────────┐
│ PREPARE MEASUREMENT TARGET            │  S2
│ OBJECT                                │
└──────────────────────────────────────┘
       │
┌──────────────────────────────────────┐
│ ENTER AND DETECT TERAHERTZ WAVE       │  S3
└──────────────────────────────────────┘
       │
┌──────────────────────────────────────┐
│ ACQUIRE FREQUENCY                     │  S4
│ CHARACTERISTIC OF MEASUREMENT         │
│ TARGET OBJECT                         │
└──────────────────────────────────────┘
       │
```

S5
FREQUENCY CHARACTERISTIC HAVING FIRST PEAK AT FIRST FREQUENCY EXISTS?

NO →

S7
DETERMINE THAT ANHYDRIDE IS NOT CONTAINED

YES ↓

S6
DETERMINE THAT ANHYDRIDE IS CONTAINED

S8
FREQUENCY CHARACTERISTIC HAVING SECOND PEAK AT SECOND FREQUENCY EXISTS?

NO →

S10
DETERMINE THAT HYDRATE IS NOT CONTAINED

YES ↓

S9
DETERMINE THAT HYDRATE IS CONTAINED

S11
FREQUENCY CHARACTERISTIC HAVING THIRD PEAK AT THIRD FREQUENCY EXISTS?

NO →

S13
DETERMINE THAT INTERMEDIATE IS NOT CONTAINED

YES ↓

S12
DETERMINE THAT INTERMEDIATE IS CONTAINED

( END )

Fig.4

EP 4 414 689 A1

Fig.5

## Fig.6

(a)

(b)

# Fig.7

| SET | SUBSTANCE AMOUNT (mmol) | MOISTURE AMOUNT (mmol) | MOISTURE AMOUNT/ SUBSTANCE AMOUNT |
|---|---|---|---|
| 1 | 1.54 | 3.21 | 2.08 |
| 2 | 0.052 | 0.104 | 2.07 |

# Fig.8

| SET | SUBSTANCE AMOUNT (mmol) | MOISTURE AMOUNT (mmol) | MOISTURE AMOUNT/ SUBSTANCE AMOUNT |
|---|---|---|---|
| 1 | 6.54 | 6.57 | 1.00 |
| 2 | 4.79 | 6.27 | 1.31 |
| 3 | 4.86 | 6.15 | 1.27 |
| 4 | 0.67 | 0.65 | 0.971 |

*Fig.9*

(a)

ELAPSE OF TIME

(b)

ELAPSE OF TIME

(c)

*Fig.10* (a)

ELAPSE OF TIME

(b)

ELAPSE OF TIME

(c)

**Fig.11**

*Fig.12* (a)

ELAPSE OF TIME

(b)

ELAPSE OF TIME

(c)

# Fig.13

START

PREPARE MEASUREMENT TARGET OBJECT ～S21

ENTER AND DETECT TERAHERTZ WAVE ～S22

ACQUIRE FREQUENCY CHARACTERISTIC OF MEASUREMENT TARGET OBJECT ～S23

S24

HAVE PEAK AT 2.65 THz TO 2.95 THz? —NO

YES S25

S26

DETERMINE THAT INTERMEDIATE IS CONTAINED

DETERMINE THAT INTERMEDIATE IS NOT CONTAINED

S27

HAVE PEAK AT 1.7 THz TO 1.9 THz OR 3.25 THz TO 3.45 THz? NO

YES S28

S29

DETERMINE THAT ANHYDRIDE IS CONTAINED

DETERMINE THAT ANHYDRIDE IS NOT CONTAINED

S30

HAVE PEAK AT 3.1 THz TO 3.25 THz? NO

YES S31

S32

DETERMINE THAT HYDRATE IS CONTAINED

DETERMINE THAT HYDRATE IS NOT CONTAINED

END

Fig.14

**Fig.15**

| FREQUENCY REPRESENTATIVE VALUE(THz) | 1.4 | 1.8 | 2.8 | 3.2 | 3.3 |
|---|---|---|---|---|---|
| FREQUENCY RANGE(THz) | 1.3~1.5 | 1.7~1.9 | 2.65~2.95 | 3.1~3.25 | 3.25~3.45 |
| ANHYDRIDE | × | ○ | × | × | ○ |
| INTERMEDIATE | ○ | × | ○ | × | × |
| HYDRATE | ○ | × | × | ○ | × |

*Fig.16*

EP 4 414 689 A1

# Fig.17

## Fig.18

# *Fig.19*

(a)

(b)

# *Fig.20*

```
                    ┌─────────┐
                    │  START  │
                    └─────────┘
                         │
    ┌────────────────────────────────────────────┐
    │   ACQUIRE FREQUENCY CHARACTERISTIC          │──S41
    │   OF ANHYDRIDE AND FREQUENCY                │
    │   CHARACTERISTIC OF HYDRATE                 │
    └────────────────────────────────────────────┘
                         │
    ┌────────────────────────────────────────────┐
    │   PREPARE MEASUREMENT TARGET OBJECT         │──S42
    └────────────────────────────────────────────┘
                         │
    ┌────────────────────────────────────────────┐
    │   ENTER AND DETECT TERAHERTZ WAVE           │──S43
    └────────────────────────────────────────────┘
                         │
    ┌────────────────────────────────────────────┐
    │   ACQUIRE FREQUENCY CHARACTERISTIC OF       │──S44
    │   MEASUREMENT TARGET OBJECT                 │
    └────────────────────────────────────────────┘
                         │
                                          S45
              AT LEAST
            TWO FREQUENCY
    CHARACTERISTICS HAVING PEAKS          NO
      AT DIFFERENT FREQUENCIES
         FROM EACH OTHER
             EXIST?
                         │YES
    ┌────────────────────────────────────────────┐
    │  DETERMINE THAT HYDRATION REACTION OR       │──S46
    │  DEHYDRATION REACTION PROCEEDED             │
    └────────────────────────────────────────────┘
                         │
                    ┌─────────┐
                    │   END   │
                    └─────────┘
```

## INTERNATIONAL SEARCH REPORT

| International application No. |
|---|
| **PCT/JP2022/040011** |

**A.    CLASSIFICATION OF SUBJECT MATTER**

*G01N 21/3581*(2014.01)i
FI:   G01N21/3581

According to International Patent Classification (IPC) or to both national classification and IPC

**B.    FIELDS SEARCHED**

Minimum documentation searched (classification system followed by classification symbols)

G01N21/00-21/01, G01N21/17-21/61

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

ACS Publications

**C.    DOCUMENTS CONSIDERED TO BE RELEVANT**

| Category* | Citation of document, with indication, where appropriate, of the relevant passages | Relevant to claim No. |
|---|---|---|
| Y | JP 2013-152159 A (HAMAMATSU PHOTONICS KABUSHIKI KAISHA) 08 August 2013 (2013-08-08) <br> paragraphs [0004]-[0060], fig. 1-7 | 1-9 |
| Y | WO 2009/041504 A1 (ARKRAY, INC.) 02 April 2009 (2009-04-02) <br> paragraphs [0006], [0007], [0033]-[0053], fig. 3-13 | 1-9 |
| Y | BRAUN, Doris E. et al. The Complexity of Hydration of Phloroglucinol: A Comprehensive Structural and Thermodynamic Characterization. THE JOURNAL OF PHYSICAL CHEMISTRY B, 05 March 2012, Vol. 116, pp. 3961-3972, <DOI: 10.1021/jp211948q> <br> p. 3961, right column, lines 19-21, fig. 1 | 5-9 |
| Y | JP 2015-179068 A (CANON KABUSHIKI KAISHA) 08 October 2015 (2015-10-08) <br> paragraph [0059] | 9 |
| A | JP 2008-164594 A (RIKAGAKU KENKYUSHO) 17 July 2008 (2008-07-17) <br> paragraphs [0054]-[0057], fig. 8 | 1-9 |
| A | JP 2008-224449 A (HAMAMATSU PHOTONICS KK) 25 September 2008 (2008-09-25) <br> paragraphs [0001]-[0079], fig. 1-14 | 1-9 |

☑ Further documents are listed in the continuation of Box C.    ☑ See patent family annex.

| | |
|---|---|
| *    Special categories of cited documents: <br> "A"  document defining the general state of the art which is not considered to be of particular relevance <br> "E"  earlier application or patent but published on or after the international filing date <br> "L"  document which may throw doubts on priority claim(s) or which is cited to establish the publication date of another citation or other special reason (as specified) <br> "O"  document referring to an oral disclosure, use, exhibition or other means <br> "P"  document published prior to the international filing date but later than the priority date claimed | "T"  later document published after the international filing date or priority date and not in conflict with the application but cited to understand the principle or theory underlying the invention <br> "X"  document of particular relevance; the claimed invention cannot be considered novel or cannot be considered to involve an inventive step when the document is taken alone <br> "Y"  document of particular relevance; the claimed invention cannot be considered to involve an inventive step when the document is combined with one or more other such documents, such combination being obvious to a person skilled in the art <br> "&"  document member of the same patent family |

| Date of the actual completion of the international search | Date of mailing of the international search report |
|---|---|
| **06 December 2022** | **20 December 2022** |

| Name and mailing address of the ISA/JP | Authorized officer |
|---|---|
| **Japan Patent Office (ISA/JP)** <br> **3-4-3 Kasumigaseki, Chiyoda-ku, Tokyo 100-8915** <br> **Japan** | |
| | Telephone No. |

Form PCT/ISA/210 (second sheet) (January 2015)

## INTERNATIONAL SEARCH REPORT

International application No.

**PCT/JP2022/040011**

### C. DOCUMENTS CONSIDERED TO BE RELEVANT

| Category* | Citation of document, with indication, where appropriate, of the relevant passages | Relevant to claim No. |
|---|---|---|
| A | BAXTER, Jason B. et al. Terahertz Spectroscopy, Analytical Chemistry, 02 May 2011, vol. 83, pp. 4342-4368, <DOI: 10.1021/ac200907z><br>Introduction to THz Spectroscopy, Attenuated Total Reflection (ATR), Liquids, Solids, Biomolecules | 1-9 |
| A | BALBUENA, Perla B. et al. Vibrational Spectra of Anhydrous and Monohydrated Caffeine and Theophylline Molecules and Crystals. The Journal of Physical Chemistry A, 25 September 2008, vol. 112, pp. 10210-10219, <DOI: 10.1021/jp805499m><br>3.2.2 MD Results, fig. 9 | 1-9 |
| A | SAKAI, Saburo et al. Pulsed Terahertz Radiation for Sensitive Quantification of Carbonate Minerals, ACS OMEGA, 05 February 2019, vol. 4, pp. 2702-2707, <DOI: 10.1021/acsomega.8b03311><br>ABSTRACT, 1. INTRODUCTION, 2.1. Absorbance and Reflective Index of Ca-Mg Carbonate Minerals. | 1-9 |

Form PCT/ISA/210 (second sheet) (January 2015)

**INTERNATIONAL SEARCH REPORT**
Information on patent family members

International application No.

**PCT/JP2022/040011**

| Patent document cited in search report | | | Publication date (day/month/year) | Patent family member(s) | | | Publication date (day/month/year) |
|---|---|---|---|---|---|---|---|
| JP | 2013-152159 | A | 08 August 2013 | US | 2013/0187050 | A1 | |
| | | | | paragraphs [0005]-[0066], fig. 1-7 | | | |
| | | | | GB | 2498853 | A | |
| WO | 2009/041504 | A1 | 02 April 2009 | US | 2010/0243901 | A1 | |
| | | | | paragraphs [0006], [0007], [0047]-[0066], fig. 3-13 | | | |
| | | | | EP | 2199778 | A1 | |
| | | | | CN | 101802593 | A | |
| JP | 2015-179068 | A | 08 October 2015 | US | 2015/0241348 | A1 | |
| | | | | paragraph [0063] | | | |
| JP | 2008-164594 | A | 17 July 2008 | US | 2008/0137068 | A1 | |
| | | | | paragraphs [0077]-[0080], fig. 8 | | | |
| | | | | EP | 1930714 | A2 | |
| | | | | CN | 101196467 | A | |
| JP | 2008-224449 | A | 25 September 2008 | US | 2010/0091266 | A1 | |
| | | | | paragraphs [0001]-[0094], fig. 1-14 | | | |
| | | | | WO | 2008/111351 | A1 | |
| | | | | EP | 2128600 | A1 | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **DORI E. BRAUN.** The Complexity of Hydration of Phloroglucinol: A Comprehensive Structural and Thermodynamic Characterization. *THE JOURNAL OF PHYSICAL CHEMISTRY B,* 2012, vol. 116, 3961-3972